# EUROPEAN PATENT APPLICATION

(11) **EP 4 759 271 A1**
(43) Date of publication of application: **17.06.2026**
(21) Application number: 25222244.3
(22) Date of filing: 10.12.2025
(51) Int. Cl.: A61F 2/44, A61F 2/46

(54) **ADJUSTABLE LORDOSIS EXPANDABLE IMPLANTS AND INSTRUMENTS**

(30) Priority: 10.12.2024 US 202418974945
(71) Applicant: Globus Medical, Inc., Audubon, PA 19403 (US)
(72) Inventor: GAMBOGI, Julia, EAGLEVILLE, 19403 (US); BAKEY, Matthew, PHILADELPHIA, 19130 (US); WEIMAN, Mark, DOWNINGTOWN, 19335 (US)
(74) Representative: Morabito, Sara

(57) **Abstract**

Expandable fusion devices, systems, instruments, and methods thereof. The expandable fusion implant may include upper and lower endplates configured to engage adjacent vertebrae and an actuator assembly for expanding the upper and lower endplates. An insertion and expansion instrument includes outer and inner drivers, which allow the surgeon to independently control parallel and lordotic expansion of the implant.

## Description

### FIELD OF THE INVENTION

The present disclosure generally relates to devices and methods for promoting an intervertebral fusion, and more particularly relates to expandable fusion devices capable of being inserted between adjacent vertebrae to facilitate the fusion process and related systems, instruments, and methods.

### BACKGROUND OF THE INVENTION

A common procedure for handling pain associated with intervertebral discs that have become degenerated due to various factors, such as trauma or aging, is the use of intervertebral fusion devices for fusing one or more adjacent vertebral bodies. Generally, to fuse the adjacent vertebral bodies, the intervertebral disc is first partially or fully removed. An intervertebral fusion device is then inserted between neighboring vertebrae to maintain normal disc spacing and restore spinal stability, thereby facilitating an intervertebral fusion.

There are a number of fusion devices and methodologies for accomplishing the intervertebral fusion. These may include solid bone implants, fusion devices which include a cage or other implant mechanism, which may be packed with bone and/or bone growth inducing substances, and expandable implants. The expandable implants may be inserted into the intervertebral disc space at a minimized height, and then expanded to restore height loss in the disc space.

There are drawbacks, however, with existing expandable implants including excessive impaction during insertion, visual obstruction, and imperfect matching with patient's lordosis due to discrete increments in lordotic angulation. As such, there exists a need for fusion devices capable of providing distraction as well as achieving optimal height restoration and changes in lordotic angulation independently from its expansion.

### SUMMARY OF THE INVENTION

To meet this and other needs, implants, systems, instruments, and methods for performing intervertebral fusion and spine stabilization are provided. In particular, expandable intervertebral implants, for example, for anterior spinal surgery may be used to treat a variety of patient indications. The expandable implants are configured to be inserted into the intervertebral disc space at a minimized height, and then expanded axially to restore height loss in the disc space, obtain normal spinal alignment, and/or distribute the load across the vertebral endplates. The implant may provide distraction as well as achieving optimal height restoration. The implant may also change in lordotic angulation independently from its expansion.

According to one embodiment, an expandable implant includes an upper endplate and a lower endplate configured to engage adjacent vertebrae, and an actuator assembly for expanding the upper and lower endplates to independently control anterior and posterior heights of the implant. The actuator assembly includes a moveable anterior actuator, a moveable posterior actuator, and a stationary posterior actuator positioned between the upper and lower endplates, an actuator screw threads into an anterior actuator nut located in the moveable anterior actuator and a posterior actuator nut located in the stationary posterior actuator. The actuator screw threads through the moveable posterior actuator to translate the posterior actuator. The upper and lower endplates interface with the moveable anterior actuator, moveable posterior actuator, and stationary posterior at complimentary mating ramps that slidably engage with one another to allow for expansion of the upper and lower endplates. The mating ramps include single point of contact ramps and full contact ramps. When the actuator screw and anterior actuator nut are turned together, the moveable posterior actuator moves toward the stationary posterior actuator, forcing the upper and lower endplates apart resulting in parallel expansion, and when only the anterior actuator nut is turned, the moveable anterior actuator moves alone to increase the anterior height, resulting in an increase in lordotic angle.

The expandable implant may include one or more of the following features. The single point of contact ramps may include a single point or edge of contact along one ramp surface that makes contact with the corresponding ramp surface. The full contact ramps may include constant continuous contact between the ramped surfaces, thereby carrying a majority of lifting force during expansion. The single point of contact ramps may be located near lateral sides of the implant and the full contact ramps may be located in between the single point of contact ramps close to the midline of the implant. The anterior actuator may define a pair of pin holes configured to receive limit pins that retain the anterior actuator nut in the anterior actuator. The pin holes may define non-threaded vertical slots that are generally perpendicular to a longitudinal axis of the implant. The limit pins may include a solid body with an L-shaped notched end and an enlarged base at the opposite end. The limit pins may limit the travel of the anterior actuator assembly by contacting surfaces on the upper and lower endplates when the implant is at its maximum expansion or lordosis.

According to another embodiment, an insertion and expansion instrument may include an insertion sub-assembly, an outer driver sub-assembly, an expansion sub-assembly, and a torque limiting handle. The insertion sub-assembly is configured to attach directly to an implant. The insertion sub-assembly has an outer tube with a threaded rod and non-threaded rod on opposite sides of the outer tube, and an insertion collar for receiving the outer tube, threaded rod, and non-threaded rod. The outer driver sub-assembly has an outer driver positionable through the outer tube and a drive collar that attaches to the outer tube. The expansion sub-assembly has an inner driver positionable through the outer driver and a housing with a removable cap containing an internal expansion mechanism configured to control both the inner driver and the outer driver. The internal expansion mechanism includes an inner shaft configured to engage with the inner driver, an outer driver gear configured to engage with the outer driver, an input gear which transmits torque to the inner shaft and/or outer driver gear, and a switch assembly that switches between parallel and lordotic expansion modes. The torque limiting handle is configured to transmit torque to the input gear.

The insertion and expansion instrument may include one or more of the following features. In parallel expansion mode, the expansion mechanism rotates the inner driver and outer driver simultaneously, and in lordotic expansion mode, only the outer driver rotates while the inner driver remains stationary. The internal expansion mechanism may include a parallel expansion gear defining teeth, an anti-rotation gear defining teeth, and the inner shaft includes a toothed member with front-facing teeth configured to interface with the teeth of the anti-rotation gear and rear-facing teeth configured to interface with the teeth of the parallel expansion gear. The switch assembly may include a switch, a mounting plate attached to the housing, a switch casing positioned in the mounting plate and having an elongated slot, and a pin connecting the switch to the elongated slot of the switch casing. When the switch is flipped, the switch casing travels to move the parallel expansion gear and the anti-rotation gear into or out of engagement with the toothed member of inner shaft. The internal expansion mechanism may include a parallel gear shaft aligned in parallel to the inner shaft, the parallel gear shaft includes spur gears at each end, the input gear includes a spur gear which interfaces with the spur gear at one end of the parallel gear shaft, and the outer driver gear includes a spur gear which interfaces with the spur gear at the other end of the parallel gear shaft to thereby transfer motion from the input shaft, through the parallel gear shaft, and to the outer driver gear. A parallel expansion indicator may be positioned along a threaded portion of the inner shaft, and a lordotic expansion indicator may be positioned along the outer driver gear. The housing may define a pair of windows to view travel of the respective indicators, thereby representing an amount of parallel and/or lordotic expansion. The inner and outer drivers may be spring-loaded forward in order to maintain engagement with the implant. The drive collar may define four notches at 90-degree intervals, which are configured to interface with pins on the outer tube of the insertion sub-assembly, to thereby lock the implant into one of four possible offset orientations. The outer tube may include a proximal plate defining a pair of bores and a distal plate defining a pair of bores, wherein the respective bores secure the threaded and non-threaded rods to the outer tube, and the distal plate includes a distal face configured to contact the implant when distal ends of the threaded and non-threaded rods engage with the implant.

According to another embodiment, a method of spinal fixation may include: (a) attaching an insertion and expansion instrument to an expandable intervertebral implant, the expandable intervertebral implant includes upper and lower endplates and an actuator assembly including a moveable anterior actuator, a moveable posterior actuator, and a stationary posterior actuator positioned between the upper and lower endplates, an actuator screw threads into an anterior actuator nut located in the moveable anterior actuator and a posterior actuator nut located in the stationary posterior actuator, the actuator screw threads through the moveable posterior actuator to translate the moveable posterior actuator, and the insertion and expansion instrument includes an insertion sub-assembly having an outer tube with a threaded rod and a non-threaded rod on opposite sides of the outer tube, which engage with the moveable anterior actuator, an outer driver sub-assembly having an outer driver positionable through the outer tube, an expansion sub-assembly having an inner driver positionable through the outer driver and a housing with a removable cap containing an internal expansion mechanism configured to control both the inner driver and the outer driver, and a torque limiting handle configured to transmit torque to the internal expansion mechanism; (b) inserting the expandable intervertebral implant into a disc space between adjacent vertebrae; and (c) expanding the implant by engaging the outer driver with the anterior actuator nut and the inner driver with the actuator screw and via the insertion and expansion instrument either: (1) rotating the inner driver and outer driver simultaneously to rotate the actuator screw and the anterior actuator nut together to expand the upper and lower endplates in parallel expansion; or (2) rotating only the outer driver to rotate the anterior actuator nut to expand the upper and lower endplates in lordotic expansion. The inner driver and outer driver may rotate simultaneously in the same direction to turn the actuator screw and anterior actuator nut simultaneously. The method may also include inserting bone anchors through sockets in the upper and lower endplates and into the adjacent vertebrae, and rotating blocking screws in the upper and lower endplates to cover the bone anchors and prevent the bone anchors from backing out.

According to yet another embodiment, a kit may include a plurality of expandable implants of different sizes and configurations, fasteners or anchors, k-wires, and other components for performing the procedure. The kit may further include one or more instruments suitable for installing and/or removing the implants, such as insertion devices or drivers, expansion instruments, removal devices, and other tools and devices which may be suitable for surgery.

### BRIEF DESCRIPTION OF THE DRAWINGS

The present embodiments will become more fully understood from the detailed description and the accompanying drawings, wherein:
FIGS. 1A-1C illustrate perspective views of an expandable implant in a fully collapsed position, at maximum expansion, and at maximum lordosis, respectively, according to one embodiment;
FIGS. 2A-2C illustrate side views of the expandable implant in the fully collapsed position, at maximum expansion, and at maximum lordosis, respectively, according to one embodiment;
FIGS. 3A-3C illustrate cross-sectional views of the expandable implant in the fully collapsed position, at maximum expansion, and at maximum lordosis, respectively, according to one embodiment;
FIG. 4 illustrates an exploded view of the expandable implant according to one embodiment;
FIGS. 5A-5B illustrate a single point of contact between the ramps of the expandable implant according to one embodiment;
FIGS. 6A-6B illustrate fully collapsed and fully expanded anterior views of the implant, respectively, highlighting the midline full contact ramps and lateral single point of contact ramps, respectively, according to one embodiment;
FIGS. 7A-7B show a top view and axial cross-sectional view, respectively, of the expandable implant according to one embodiment;
FIG. 8 shows a partial view of the expandable implant where the limit pins contact the endplates according to one embodiment;
FIGS. 9A-9C illustrate side and exploded views, respectively, of an insertion and expansion instrument according to one embodiment;
FIG. 10 shows an exploded view of the insertion sub-assembly according to one embodiment;
FIGS. 11A-11B show assembled and close-up distal views, respectively, of the insertion sub-assembly;
FIGS. 12A-12B show top and cross-sectional views, respectively, of the insertion sub-assembly;
FIGS. 13A-13C show side views of the insertion sub-assembly with attachment to the implant in collapsed and expanded positions, respectively;
FIG. 14 shows an exploded view of the outer driver sub-assembly according to one embodiment;
FIGS. 15A-15B show assembled and cross-sectional views, respectively, of the driver subassembly;
FIGS. 16A-16C show close-up cross-sectional and perspective views, respectively, of the drive collar assembly according to one embodiment;
FIG. 17 shows an exploded view of the expansion driver sub-assembly according to one embodiment;
FIG. 18A-18C show side views of the expansion driver sub-assembly including the removable cap and the indicators for lordosis and anterior expansion according to one embodiment;
FIGS. 19A-19B shows close-up assembled and cross-sectional views, respectively, of the housing portion and expansion mechanisms of the expansion driver sub-assembly according to one embodiment; and
FIGS. 20A-20B show relative movement of the inner and outer drivers for parallel expansion and lordosis, respectively, according to one embodiment.

### DETAILED DESCRIPTION

Embodiments of the disclosure are generally directed to devices, systems, instruments, and methods for intervertebral fusion and spine stabilization. Specifically, expandable implants are configured to be inserted into the intervertebral disc space at a minimized height, and then expanded axially to restore height loss, provide normal spinal alignment, and/or distribute the load across the vertebral endplates. The implant may provide distraction as well as achieving optimal height restoration. The implant may also change in lordotic angulation independently from its height expansion.

A spinal fusion is typically employed to eliminate pain caused by motion of degenerated disc material. Upon successful fusion, a fusion device becomes permanently fixed within the intervertebral disc space. The expandable fusion device may be positioned between adjacent vertebral bodies in a collapsed position. The expandable fusion device is configured to expand in height to restore height loss in the disc space. The fusion device engages the endplates of the adjacent vertebral bodies and, in the installed position, maintains desired intervertebral disc spacing and restores spinal stability, thereby facilitating the intervertebral fusion.

Minimally invasive surgery (MIS) may be used to preserve muscular anatomy by only causing disruption where necessary. The benefit of the MIS surgical approach is that it can reduce post-operative pain and improve recovery time for patients. In one embodiment, the expandable fusion device can be configured to be placed down an endoscopic tube and into the surgical target site. By way of example, the surgical site may be an intervertebral disc space situated between two adjacent vertebrae. Although particularly suited for use in an anterior lumbar interbody fusion (ALIF), it will be readily appreciated by those skilled in the art that the implant may be employed in any number of suitable orthopedic approaches and procedures, such as direct lateral where coronal deformity is encountered. Other approaches may include but are not limited to posterior, lateral, anterolateral, posterolateral, or transforaminal approaches to the lumbar spine, cervical spine, or thoracic spine, as well as any non-spine application, such as treatment of bone fractures and the like. The terms implant, interbody, interbody implant, fusion device, spacer, and expandable device may be used interchangeably herein.

Components of all of the devices disclosed herein may be manufactured of any suitable materials including metals (e.g., titanium), metal alloys (e.g., stainless steel, cobalt-chromium, and titanium alloys), ceramics, plastics, plastic composites, or polymeric materials (e.g., polyether ether ketone (PEEK), polyphenylene sulfone (PPSU), polysulfone (PSU), polycarbonate (PC), polyetherimide (PEI), polypropylene (PP), polyacetals, or mixtures or co-polymers thereof), and/or combinations thereof. In some embodiments, the devices may include radiolucent and/or radiopaque materials. The components can also be machined and/or manufactured using any suitable techniques (e.g., 3D printing).

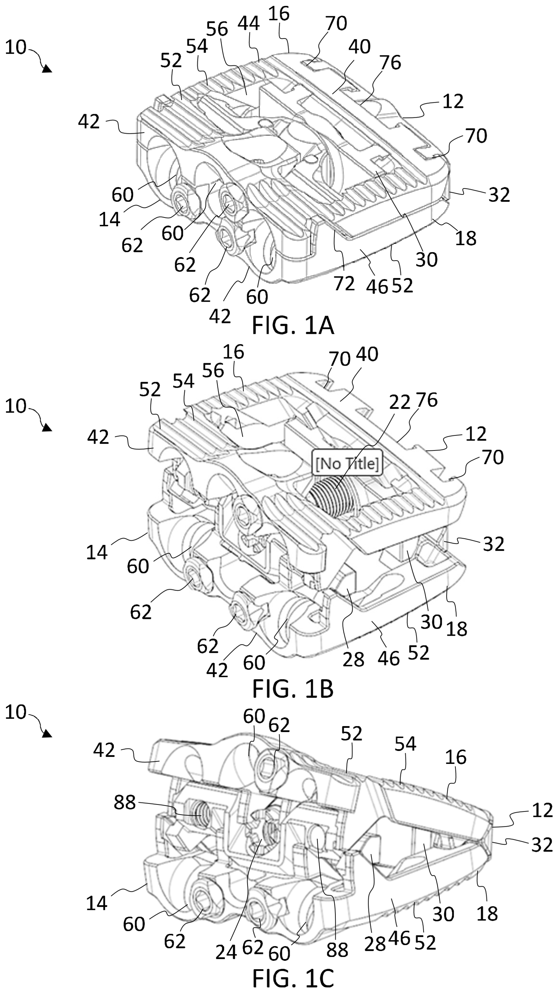

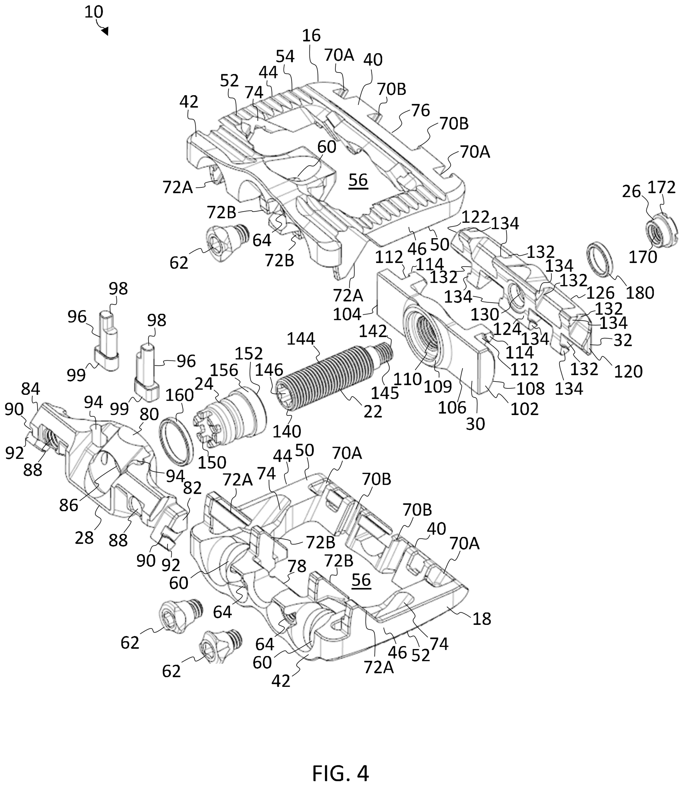

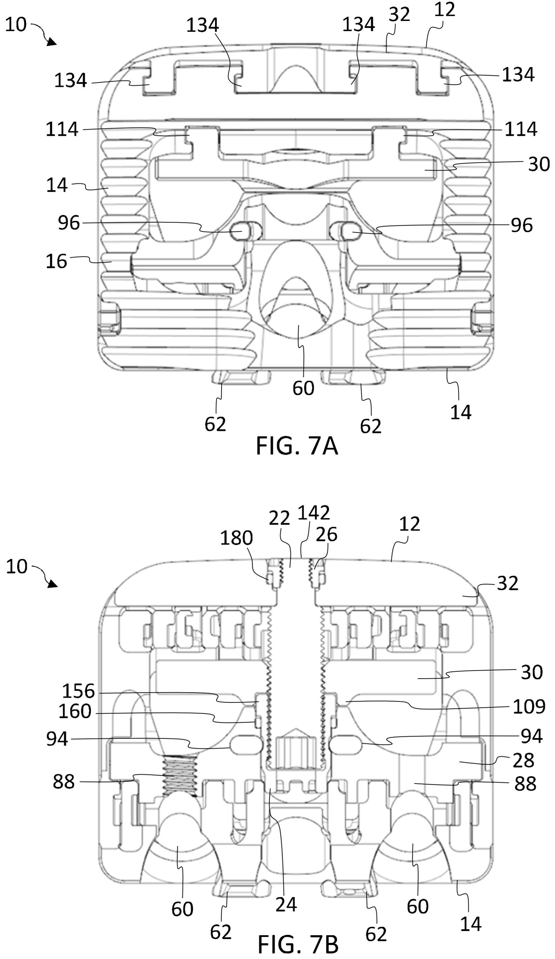

Turning now to the drawing, where like reference numerals may refer to like elements, FIG. 1A-8 illustrate an expandable fusion device or implant 10 according to one embodiment. The expandable implant 10 extends along a central longitudinal axis A between a front end or posterior end 12 and a rear end or anterior end 14 of the device 10. The implant 10 includes upper and lower endplates 16, 18 configured to engage adjacent vertebrae, which define a height of the implant 10. The implant 10 includes an actuator assembly 20 configured for expanding the upper and lower endplates 16, 18. The actuator assembly 20 includes a single actuator screw 22 threaded into anterior and posterior actuator ramps 28, 30 to independently control the anterior and posterior height of the implant 10.

The implant 10 may include three separate actuators 28, 30, 32 positioned between the upper and lower endplates 16, 18: a moveable anterior actuator 28, a moveable posterior actuator 30, and a stationary posterior actuator 32. One end of the actuator screw 22 threads into an anterior actuator nut 24 located in the anterior actuator 28 and the opposite end of the actuator screw 22 threads into a posterior actuator nut 26 affixed to the stationary posterior actuator 32. The actuator screw 22 threads through the moveable posterior actuator 30 to translate the posterior actuator 30 along central longitudinal axis A. When the actuator screw 22 and anterior actuator nut 24 are turned together, the moveable posterior actuator 30 moves toward the stationary posterior actuator 32, forcing the upper and lower endplates 16, 18 apart resulting in parallel expansion. When only the anterior actuator nut 24 is turned, the moveable anterior actuator 28 moves alone to increase the anterior height, resulting in an increase in lordotic angle.

The upper and lower endplates 16, 18 interface with the actuators 28, 30, 32 via a plurality of mating ramps 36, 38. The complimentary mating ramps 36, 38 may include angled, inclined, or sloped surfaces that slidably interface with one another to allow for expansion of the upper and lower endplates 16, 18. The mating ramps 36, 38 allow for controlled, sliding movement on the complementary surfaces, thereby providing vertical displacement of endplates 16, 18. The mating ramps 36, 38 may include single point of contact ramps 36 and full contact ramps 38. The single-point and full contact ramps 36, 38 may be integrated into the same implant 10 allowing for precise adjustment of both the height and angle of the implant 10 according to the specific surgical needs.

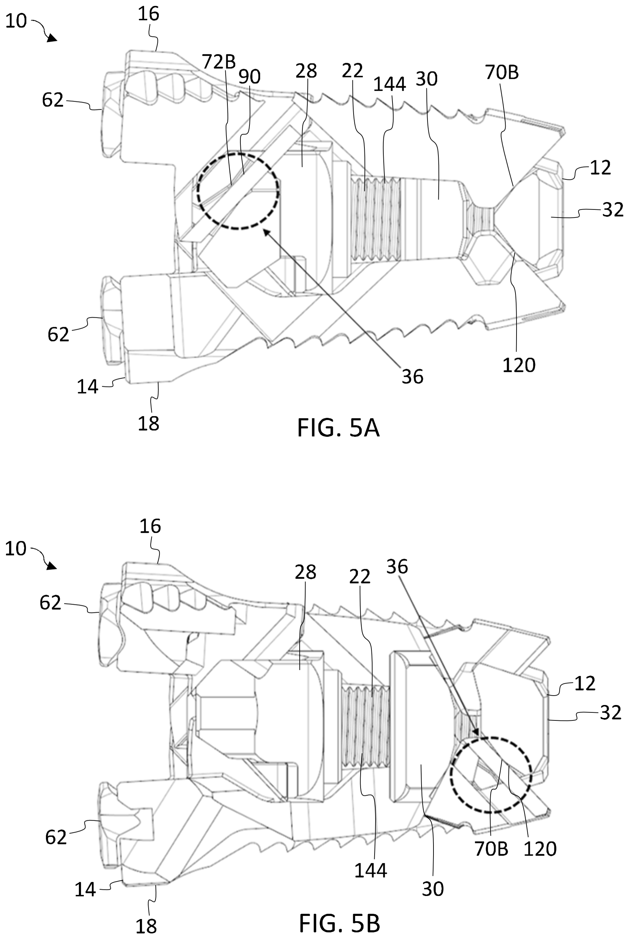

As best seen in FIGS. 5A-5B, the single point of contact ramps 36 may involve a ramp design where a localized contact area, such as a single point, edge, or minimal surface area along one ramp surface makes contact with the corresponding ramp surface at any given time during the actuation. The single point of contact ramps 36 may allow for controlled slidable, articulated, or pivotal movement around the point of contact. As the actuator(s) 28, 30, 32 move, the point of contact may shift along the corresponding ramp surface effectively changing the angle or position of the endplates 16, 18 in a controlled manner. The single point of contact ramps 36 may allow for fine-tuned adjustments in the height and/or angle of the endplates 16, 18 to achieve the desired height expansion and spinal correction.

The full contact ramps 38 may involve a ramp design where the ramp surface maintains constant continuous contact with the corresponding ramp surface throughout the actuation process. The full contact ramps 38 may carry the majority of the lifting force at a shorter moment-arm distance for the actuator screw 22, reducing bending in the ramps and improving overall lift force. In other words, the full contact ramps maintain maximum surface area contact between the ramp surfaces during the actuation, and the continuous contact distributes forces more evenly across the interface providing a stable and uniform expansion of the endplates 16, 18. The full contact ramps 38 may not have engagement tabs in order to improve manufacturability. Further, the even distribution of force reduces the risk of localized stress points and can enhance the overall stability of the spinal fixation.

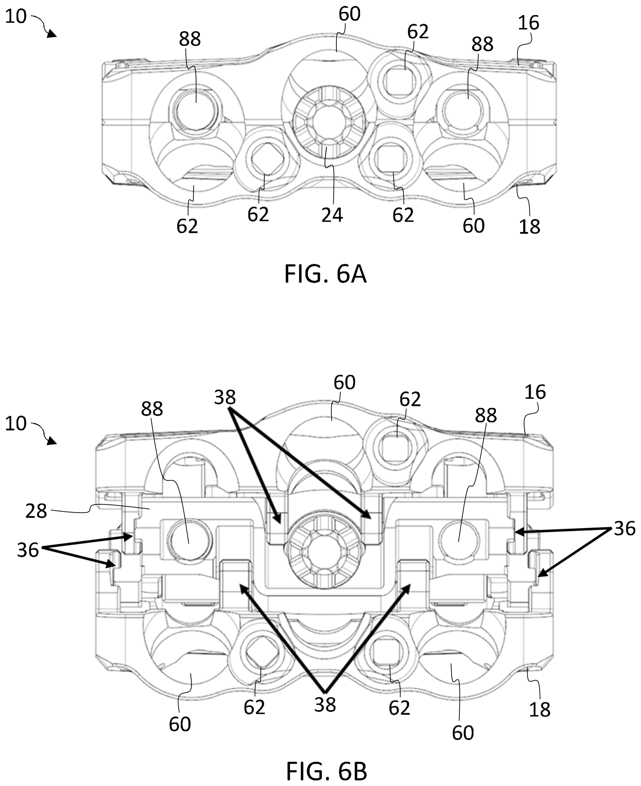

The single-point and full contact ramps 36, 38 may be distributed throughout the implant 10 to achieve targeted adjustments as well as uniform lifting during spinal correction procedures. As best seen in FIG. 6B, the single-point ramps 36 may be located along or near the lateral edges or sides of the implant 10, and the full contact ramps 38 may be located near or close to the mid-line or central axis A of the implant 10. In other words, the full contact ramps 38 may be positioned in the middle between the single-point ramps 36 at each lateral side. The single-point ramps 36 may address specific angular corrections while the full contact ramps 38 ensure that the adjustments do not compromise the overall stability of the implant 10. The full contact ramps 38 may be located close to or near the mid-line of the implant 10, thereby centralizing the lifting force where it can be most effective in supporting the structural integrity of the implant 10.

The implant 10 is configured to be inserted into the disc space in a collapsed configuration. FIG. 1A shows the implant 10 in the fully collapsed configuration. Once inserted into the disc space, the implant 10 is expanded in height to an expanded configuration to precisely restore spinal alignment and distribute load across the vertebral endplates. FIG. 1B shows the implant 10 in a fully expanded configuration with maximum expansion. FIG. 1C shows the implant 10 in an expanded configuration with maximum lordosis. It will be appreciated that the implant 10 may be expanded to any desired height between these extremes. The anterior and posterior heights are independently adjustable to a desired lordotic profile. In this manner, the height is adjustable to restore height loss in the disc space and lordotic angulation. It should be understood that reference to the front and rear ends and anterior and posterior heights are described with respect to the direction of placement into an intervertebral disc space with the front of the expandable fusion device 10 placed into the disc space first, followed by the rear of the expandable fusion device 10, and then expanding the endplates 16, 18 in height and/or lordosis. These and other directional terms may be used herein for descriptive purposes and do not limit the orientation(s) in which the devices may be used.

Each endplate 16, 18 may include a front or posterior rail 40 and a rear or anterior rail 42 extending between opposed side rails 44, 46. The rails 40-46 define an inner face 50 and an opposite outer face 52. The inner face 50 may be configured to mate with the respective actuators 28, 30, 32 and the outer face 52 may be configured to contact adjacent vertebrae. The outer face 52 of each endplate 16, 18 may include a plurality of teeth 54 or other friction increasing elements, such as ridges, roughened surfaces, keels, gripping or purchasing projections configured to retain the device 10 in the disc space. The endplates 16, 18 may be 3D printed using additive manufacturing. In this manner, the outer face 52 may be created with teeth and/or surface texturing that can better facilitate bony on-growth. Each endplate 16, 18 may define a vertical window or through passage 56, thereby defining a central graft chamber within the implant 10. The window or through passage 56 allows graft material or other therapeutically beneficial material to be packed into or grow through the implant 10.

The implant 10 may be secured to the adjacent vertebrae with one or more anchors or fixation screws (not shown). For example, the anterior rail 42 may define at least one anchor socket 60 configured for receiving the anchor or fixation screw therethrough and into the adjacent vertebra. In the embodiment shown, three sockets 60 for receiving three respective anchors or screws are provided in the upper and lower endplates 16, 18: one socket 60 pointed upward into the superior vertebra and two sockets 60 pointed downward into the inferior vertebra. The sockets 60 may be surrounded by a hemispherical recess such that the anchors or screws may be angled into the adjacent vertebrae. The bone screw holes 60 in the endplates 16, 18 may be compatible with both traditional lag screws and/or anchor fixation. In one embodiment, the bone screws may be polyaxial screws, and sockets 60 correspondingly shaped, such that the polyaxial screws may be inserted at optimal angles with respect to implant 10. In another embodiment, the anchor may be a curved, t-shaped shim-type anchor with sharp edges to penetrate bone. Examples of bone screws and anchors are further described in in U.S. Patent No. 11,554,023, which is incorporated by reference herein in its entirety for all purposes. Although a given configuration of sockets 60 is shown, it will be appreciated that the sockets 60 may be present in any suitable number and configuration for fixation. In the alternative, the sockets 60 may be omitted to provide a standalone device.

Cam style blocking screws 62 may be used to block the anchors or fixation screws from backing out after being inserted. The anterior rail 42 may define a blocking screw hole 64 positioned next to each respective socket 60. The blocking screw holes 64 may be internally threaded to receive the respective threaded blocking screws 62. In one embodiment, three blocking screws 62 thread into the endplates 16, 18 to secure the anchors or fixation screws in the three respective sockets 60. The blocking screws 62 may have an enlarged head with a drive recess and a threaded shaft. These blocking screws 62 allow for one type of blocking screw to be used across all of the implant bone screw holes 60. When the blocking screws 62 are rotated and engaged, a portion of the enlarged head covers the respective anchors or fixation screws, thereby preventing migration of the installed anchors or fixation screws.

The rails 40-46 and/or inner face 50 of each endplate 16, 18 may define one or more ramps 70, 72, 74 configured to mate with the respective actuators 28, 30, 32, thereby causing endplates 16, 18 to separate apart. For example, the endplates 16, 18 may define posterior ramps 70 along the posterior rail 40, which mate with the posterior actuators 30, 32. The endplates 16, 18 define anterior ramps 72 along the anterior rail 42 and central ramps 74 along the side rails 44, 46, which mate with the anterior actuator 28. The ramps 70, 72, 74 may include ramped surfaces, angled surfaces, or inclined planes with a given gradient or angle of slope. The ramps 70, 72, 74 may have generally straight ramped surfaces, may be curved, or may be configured in any suitable manner for slidable interface between the components. The ramps 70, 72, 74 may define male or female slide ramps configured to mate with corresponding ramps 90, 112, 132 on the actuators 28, 30, 32. In one embodiment, the ramps 70, 72, 74 on endplates 16, 18 include female ramps with grooves or slots defined therein. The groove or slots may be configured to receive a portion of complimentary ramps 90, 112, 132 of the actuators 28, 30, 32.

In one embodiment, the posterior ramps 70 include both single-point of contact ramps 70A and full contact ramps 70B. The single-point of contact ramps 70A may be located near each lateral side of the posterior rail 40. A pair of full contact ramps 70B may be located centrally between the single-point of contact ramps 70A. The full contact ramps 70B may be separated by a channel or gap 76, which receives a portion of the posterior actuator 32 when fully collapsed. The anterior ramps 72 also include both single-point of contact ramps 72A and full contact ramps 72B. The single-point of contact ramps 72A may be located near each lateral side of the anterior rail 42. A pair of full contact ramps 72B may be located centrally between the single-point of contact ramps 72A. Similarly, the central ramps 74 may include single-point of contact ramps defined along an inner surface of each side rail 44, 46.

The full contact ramps 72B may be separated by a channel or gap 78, which receives a portion of the anterior actuator 28 when fully collapsed. As best seen in FIG. 6B, the gap 78 between the full contact anterior ramps 72B on the upper endplate 16 may be smaller than the gap 78 between the full contact anterior ramps 72B on the lower endplate 18. Similarly, the spacing between the single-point of contact anterior ramps 72A on the upper endplate 16 may be closer than the spacing between the single-point of contact anterior ramps 72A on the lower endplates 18. It will be appreciated that the spacing between any of the ramps 70, 72, 74 may be selected to allow for adequate nesting of the upper and lower endplates 16, 18 when the implant 10 is collapsed, for optimal lifting strength, or any other suitable parameters. As the anterior and/or posterior actuators 28, 30, 32 translate and slide against the ramps 70, 72, 74 of the endplates 16, 18, the movement provides for expansion or contraction of the implant 10.

The actuator assembly 20 includes moveable anterior actuator 28 positioned between the upper and lower endplates 16, 18, thereby providing anterior expansion to the implant 10. The moveable anterior actuator 28 includes a laterally extending body with an enlarged central portion 80. The anterior actuator 28 extends from a first free end 82 to a second free end 84. The first and second ends 82, 84 may define an irregular cross-sectional shape, such as a polygon with facets and rounded corners. The first and second free ends 82, 84 are receivable between the side rails 44, 46 toward the anterior end 14 of the upper and lower endplate 16, 18 and the enlarged central portion 80 is positionable through the graft window 56 of the upper and lower endplates 16, 18 when in the collapsed configuration. The enlarged central portion 80 defines a central non-threaded bore 86 sized and dimensioned to receive the anterior actuator nut 24. The central axis of bore 86 may be aligned with the central longitudinal axis A of the implant 10.

Additional recesses or bores 88 may be provided through the anterior actuator 28. For example, the anterior actuator 28 may include two attachment holes 88 designed to interface with instrumentation for insertion and/or removal of the implant (e.g., threaded and non-threaded rods 224, 226 of instrument 200). A first threaded bore 88 may be provided on one side of the central bore 86 and a second non-threaded bore 88 may be provided on the opposite side of the central bore 86. Recesses or bores 88 may be used to attach an instrument, such as an insertion and/or expansion instrument 200. In addition, the implant 10 may have multiple anterior openings to mate with a bone funnel, for example, to backfill the implant in-situ after expansion.

The moveable anterior actuator 28 includes one or more ramps 90 configured to mate with corresponding anterior ramps 72, 74 on the endplates 16, 18. In one embodiment, the ramps 90 may include male ramps with projections or protrusions with a rail configured to engage with the corresponding grooves or slots in the endplates 16, 18. In some embodiments, the protrusions or rails may include L-shaped tabs 92 which extend out from the actuator 28 and then make a 90-degree turn forming the L shape. In other embodiments, the protrusion or rails may include T-shaped tabs with a stem which extends out from the actuator 28 and then provides a perpendicular extension at the free end of the stem forming the top of the T shape. In some cases, the free ends of the tabs 92 may point laterally outward and away from one another, may point inward toward one another, or may be oriented in any suitable manner. The slots machined into the upper and lower endplates 16, 18 interface with the ramps 90 on the anterior actuator 28 allowing for parallel and lordotic expansion, while preventing disassembly by pulling the endplates apart.

In one embodiment, the anterior actuator 28 may define a first pair of male ramps 90 proximate each end 82, 84 of the actuator 28 and configured to mate with the corresponding single-point of contact anterior ramps 72A along the endplates 16, 18. The single-point contact ramps 90 may be configured to interface with the corresponding anterior ramps 72A at a singular contact point or location, facilitating pivoting or rotational movement. The singular location where the ramp 90 interacts with corresponding ramp 72A may be a focal point around which rotation or movement occurs. Unlike broad surface contact between mating ramps, ramp 90 may have targeted contact at a defined point or edge that interfaces with corresponding ramp 72. For example, the engaging surface of one ramp 72B, 90 may be pointed, angled, tapered, curved, etc. to define a single point or edge of contact with the other flat or angled surface. These ramps 90 may include tabs 92 to secure the endplates 16, 18 to the anterior actuator 28 along the lateral edges of the implant 10.

The anterior actuator 28 may define a second pair of male ramps 90 toward the mid-line configured to mate with the corresponding full contact ramps 72B on the endplates 16, 18. The full contact ramps 90 may be configured to interface with the corresponding mid-line ramps 72B with continuous constant contact between the ramps 72, 90, thereby providing a lifting force at a shorter moment-arm distance for the actuator screw, reducing bending in the ramps and improving overall lift force. The midline ramps 90 on the anterior actuator 28 do not have engagement tabs to improve manufacturability, and because the endplate engagement is already achieved with the tabs 92 at the lateral edges of the part.

The anterior actuator 28 defines a pair of pin holes 94 on either side of center bore 86 configured to receive respective limit pins 96, which retain the anterior actuator nut 24 in the anterior actuator 28, while also preventing implant over-expansion. The pin holes 94 may include non-threaded vertical slots, which may be generally perpendicular to the longitudinal implant axis A. The pin holes 94 may be generally obround, oval, ellipse, or ovoid in cross-section. The limit pins 96 may include a solid body with an L-shaped or notched end 98 and an enlarged base at the opposite end 99. A portion of the pin hole 94 may have an enlarged portion configured to receive the enlarged base end 99 of the limit pin 96.

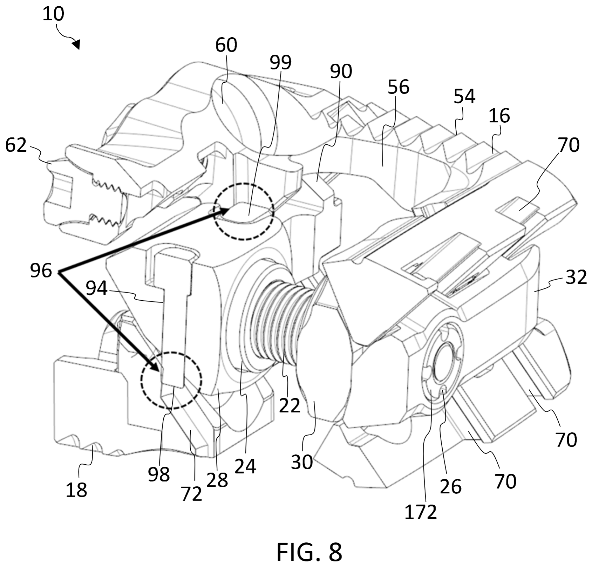

The limit pins 96 retain the anterior actuator nut 24 in the anterior actuator 28 and also function to prevent the implant 10 from over-expanding and disassembling. The limit pins 96 limit the travel of the anterior actuator assembly by contacting surfaces on the upper and lower endplates 16, 18 when the implant 10 is at its maximum lordotic expansion for the given amount of posterior expansion. For example, the limit pins 96 may allow for up to 30 degrees of endplate lordosis with 0 mm posterior expansion, and 12-14 degrees of lordosis when at maximum expansion. As best seen in the partial cut-away of FIG. 8, the limit pins 96 may contact both endplates 16, 18 simultaneously to prevent rotation of the anterior actuator 28 when at max expansion or lordosis. In addition, the limit pins 96 may contact both endplates simultaneously to provide consistent maximum expansion and prevent damage to the implant 10 when torqued-out at maximum expansion. The limit pins 96 and support surfaces on the anterior actuator 28 and endplates 16, 18 are configured to be strong enough to withstand the maximum expansion torque of the instrumentation.

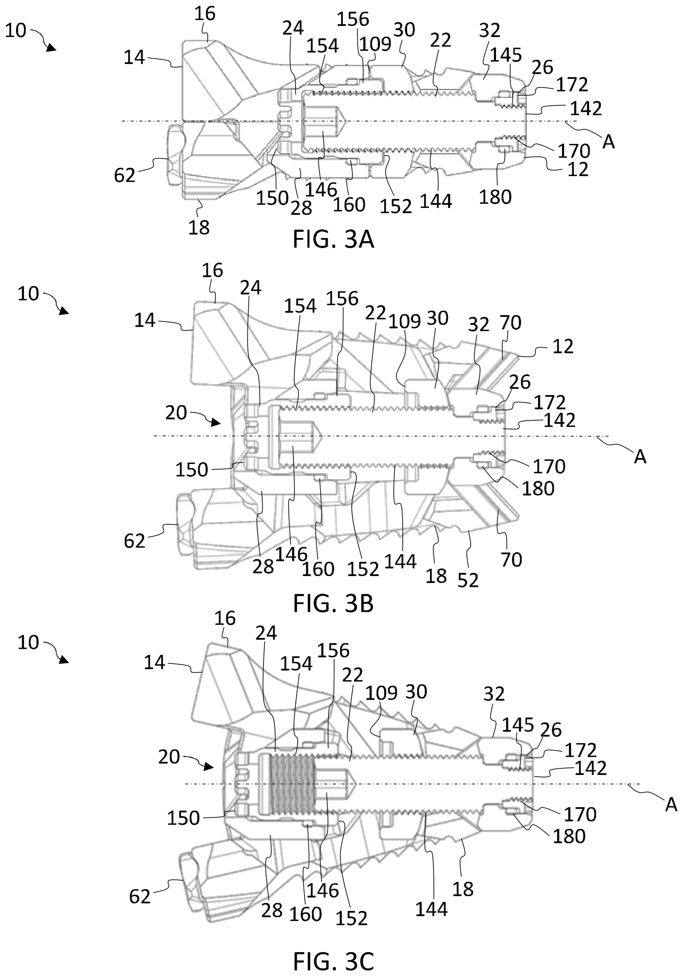

The actuator assembly 20 includes a moveable posterior actuator 30 positioned between the upper and lower endplates 16, 18, thereby providing posterior expansion to the implant 10. The moveable posterior actuator 30 includes a laterally extending body connecting a first free end 102 to a second free end 104 with a flat anterior face 106 and an opposite rounded or curved posterior face 108. The first and second free ends 102, 104 are receivable between the upper and lower endplates 16, 18 toward the posterior end 12 of the implant 10. The anterior face 106 includes a circular protrusion 109 that defines a central threaded cylindrical bore 110 configured to receive the actuator screw 22. The central axis of bore 110 may be aligned with the central longitudinal axis A of the implant 10. As best seen in FIG. 3A, in the collapsed position, a recess defined within the circular protrusion 109 may be configured to receive a portion of the enlarged collar 156 of the anterior actuator nut 24.

The moveable posterior actuator 30 includes one or more ramps 112 configured to mate with corresponding ramps 70 on the endplates 16, 18. The ramps 112 may extend from the posterior face 108 of the actuator 30. In some cases, similar to ramps 90, the ramps 112 may include male ramps with projections or protrusions with a rail configured to engage with the corresponding grooves or slots in the endplates 16, 18. The protrusions or rails may include L-shaped tabs 114, similar to tabs 92, which extend out from the actuator 30 and then make a 90-degree turn forming the L shape. In some cases, the free ends of the tabs 92 may point laterally outward and away from one another at each end of the actuator 30. Some of the ramps 112 may include a single point of contact ramp interface incorporated into each lateral side of the moveable posterior actuator 30. For example, two male ramps 112 may be positioned upward to interface with the upper endplate 16 and two male ramps 112 may be positioned downward to interface with the lower endplate 18. The tabs 92 interface with the corresponding slots in the upper and lower endplates 16, 18, preventing disassembly and allowing for independent expansion. In addition to the single-point contact ramps 112 toward the lateral edges, additional full contact ramps may be included toward the mid-line of the actuator 30 or in between the single-point of contact ramps 112 on the sides. The full contact midline ramps 112 on the moveable posterior actuator 30 may not include engagement tabs to simplify the manufacturing process and because the endplates 16, 18 are already secured by the tabs 92 located on the lateral sides of the actuator 30.

The actuator assembly 20 includes a stationary posterior actuator 32 positioned between the upper and lower endplates 16, 18, thereby providing posterior expansion to the implant 10. The stationary posterior actuator 32 includes a laterally extending body having a first free end 120 and a second free end 122 on opposite sides. The first and second free ends 120, 122 are receivable between the posterior rails 40 of the upper and lower endplate 16, 18, and the stationary posterior actuator 32 defines the nose or front end 12 of the implant 10. The stationary posterior actuator 32 includes an anterior face 124 and an opposite posterior face 126. A central portion of the actuator 32 may define a central non-threaded bore 130 sized and dimensioned to receive the posterior actuator nut 26. The central axis of bore 130 may be aligned with the central longitudinal axis A of the implant 10.

The stationary posterior actuator 32 includes one or more ramps 132 configured to mate with corresponding posterior ramps 70 on the endplates 16, 18. The ramps 132 may be defined into and/or extend from the anterior face 124 of the actuator 32. Similar to ramps 90, 112, the ramps 132 may include male ramps configured to mate with the posterior ramps 70 along the posterior rails 40 of the endplates 16, 18. The male ramps 132 may include projections or protrusions having a L-shaped tabs 134 configured to engage with the corresponding grooves or slots in the endplates 16, 18. The free ends of the tabs 134 may point laterally outward from the midline of the actuator 32. The ramps 132 toward the lateral edges may include single-point of contact ramps while the ramps 132 toward the midline may include full contact ramps. In this cases, all of the ramps 132 may include engagement tabs 134. The full contact ramps 132 near the midline may include tabs 134 to counteract the bending forces experience in lordotic lifting. When the moveable posterior actuator 30 moves toward the stationary posterior actuator 32, these posterior actuators 30, 32 interface with ramped surfaces 70 on the upper and lower endplates 16, 18, pushing the upper and lower endplate 16, 18 out in both directions, thereby expanding the posterior height of the implant 10.

In this case of the single-point of contact ramps, implant 10 uses a point of contact on the engagement interface itself as a pivot point for lordotic angle adjustment. The point contact engagement interface 90, 112, 132 includes enough angular clearance with the mating engagement slot to accommodate the endplate angle change required for in-situ lordotic adjustment and device assembly. The implant 10 also includes full contact interfaces to carry the majority of the lifting force, thereby reducing bending in the ramps and improving overall lift force.

The posterior expansion mechanism functions by using the actuator screw 22 to drive the moveable posterior actuator 30 toward or away from the stationary posterior actuator 32. As best seen in FIG. 3B, the moveable posterior actuator 30 translates away from anterior actuator 28 and posteriorly toward stationary posterior actuator 32. As the two posterior actuators 30, 32 move toward one another, these posterior actuators 30, 32 interface with ramped surfaces 70 on the upper and lower endplates 16, 18, pushing the upper and lower endplates 16, 18 out in both directions, expanding the posterior height of the implant 10.

The actuator assembly 20 includes actuator screw 22, anterior actuator nut 24, and posterior actuator nut 26 aligned along the central longitudinal axis A of the implant 10. The actuator screw 22 extends from a proximal end 140 to a distal end 142. The actuator screw 22 may include a shaft with a reduced diameter or stepped diameter near the distal end 142 relative to the remainder of the shaft. The shaft includes external threads(s) or an exterior threaded portion 144 extending along its length. The threaded shaft 144 may have a given diameter, handedness, thread form, thread angle, lead, pitch, etc. suitable for interfacing with the anterior actuator nut 24 (which controls movement of the anterior actuator 28) and the moveable posterior actuator 30. The reduced diameter at the distal end 142 may also include a threaded portion 145 suitable for interfacing with the posterior actuator nut 26. In this embodiment, a single type of thread profile may be used for both parallel and lordotic expansion. Unlike systems using up to three different sets of threads to engage for parallel expansion, this design achieves parallel and lordotic expansion through the interaction of one set of threads. This reduces the overall increase in lifting torque created by friction or binding of the threads.

The proximal end 140 of shaft 22 may define an instrument recess 146 configured to receive an instrument, such as inner driver 320, to rotate or actuate the actuator screw 22. The instrument recess 146 may include a tri-lobe, hex, star (e.g., Torx), or other suitable recess configured to engage with a driver instrument to apply torque to the actuator screw 22. In one embodiment, the instrument recess 146 has a Torx geometry optimized to maximize the allowable input torque to improve implant lifting capabilities. The proximal end 140 of the shaft is configured to thread into the anterior actuator nut 24 to translate the moveable anterior actuator 28. The threaded shaft 144 also threads through the moveable posterior actuator 30 to translate the moveable posterior actuator 30 along central longitudinal axis A.

The distal end 142 of the actuator screw 22 may have a reduced distal tip, for example, having a diameter less than the diameter of the threaded shaft 144. The distal tip 142 may be threaded and configured to thread into the posterior actuator nut 26 retained in the stationary posterior actuator 32. Even at full expansion, for example as best seen in FIG. 3B, the distal end 142 of the actuator screw 22 does not protrude out past the posterior edge or front nose 12 of the implant 10. Posterior protrusion of the actuator screw was a point of concern from surgeons due to the optics of having the thread rod protruding near the posterior structures of the spine. Thus, the expansion assembly 20 and operation for implant 10 eliminates the possibility of any posterior protrusion of the actuator screw 22.

The anterior actuator nut 24 has a body extending between a proximal end 150 to a distal end 152. As best seen in FIGS. 3A-3C, a central bore 154 extends through the body of the anterior actuator nut 24 from the proximal end 150 to the distal end 152. A portion of the bore 154 defines internal threads which are configured to threadably engage with the exterior threads 144 of the actuator screw 22. The proximal end 150 of the anterior actuator nut 24 defines a driver engagement recess, such as a series of notches and teeth (e.g., castle nut drive feature) or other suitable recess configured to engage with a driver instrument, such as outer driver 280, to apply torque to the anterior actuator nut 24. The outer driver castle nut drive feature geometry may be optimized to maximize the allowable input torque. Increased torque limits allow for improved implant lifting capabilities. The driver recess for the anterior actuator nut 24 may be preferably different from the driver recess 146 for the actuator screw 22. The distal end 152 of the actuator nut 24 may have an enlarged collar 156. An anterior drag ring 160, such as a PEEK washer or annular ring, may be nested against the collar 156. The anterior drag ring 160 may be captured between the anterior actuator nut 24 and anterior actuator 28 to provide frictional resistance against back-driving the anterior expansion mechanism resulting in loss of anterior height of the implant. Contrary to adding static friction to prevent implant height loss, the PEEK drag ring 160 also functions as a thrust washer to reduce dynamic expansion friction when under load. The anterior actuator nut 24 may be permanently captured inside the anterior actuator 28, for example, by limit pins 96 or another suitable mechanism.

The posterior actuator nut 26 includes a body with a central cylindrical bore 170 defined therethrough. The bore 170 defines internal threads which are configured to threadably engage with the exterior threads 145 of the distal tip 142 of the actuator screw 22. The actuator screw 22 is captured inside the stationary posterior actuator ramp 32 by threading into the posterior actuator nut 26. The end of the posterior actuator nut 26 may have an enlarged collar 172 with notches or teeth, for example, to help with assembly. A posterior drag ring 180 may be seated beneath the collar 172. The posterior drag ring 180, such as a PEEK washer or annular ring, is captured between the posterior actuator nut 26 and stationary posterior actuator ramp 32 to provide frictional resistance against back-driving the posterior expansion mechanism resulting in loss of overall expanded height of the implant 10. Contrary to adding static friction to prevent implant height loss, the PEEK drag ring 180 also functions as a thrust washer to reduce dynamic expansion friction when under load. The drag rings 160, 180 may be added between the actuator screw 22 and the stationary posterior actuator 32 and between the anterior actuator 28 and anterior actuator nut 24 to ensure that neither the anterior nor posterior actuators 28, 32 lose height during use. The drag rings 160, 180 also serve as thrust washers to improve expansion capabilities.

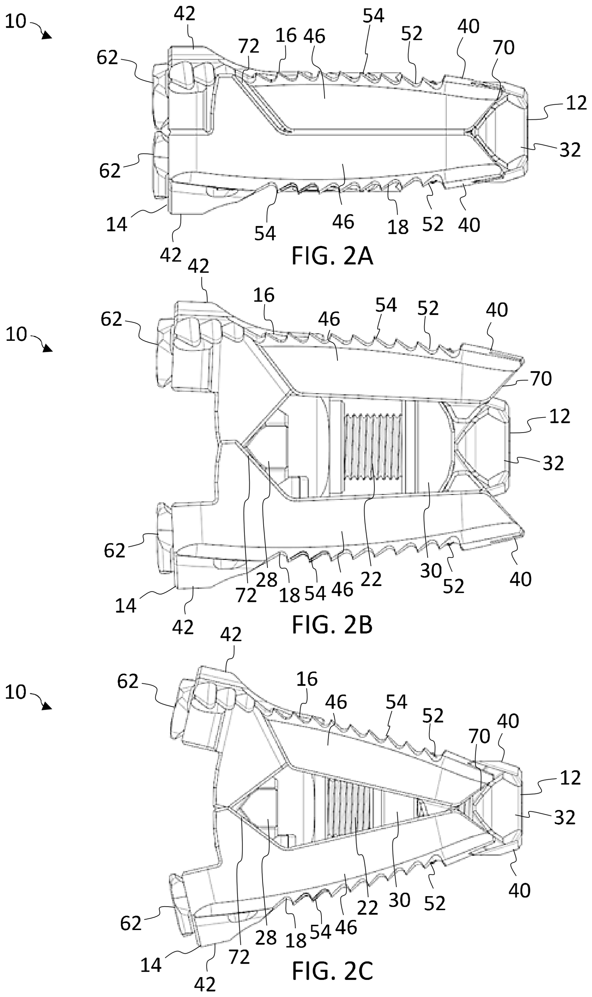

During operation, the implant 10 may be operated in one of two modes. In a first mode, the actuator screw 22 and the anterior actuator nut 24 are rotated or turned together simultaneously. This moves the moveable posterior actuator 30 toward the stationary posterior actuator 32, forcing the upper and lower endplates 16, 18 apart. Because the ramp angle of the ramps 72 on the anterior end of the endplate 16, 18 match the ramp angle of the ramps 70 on the posterior end of the endplate 16, 18, this results in equal expansion of both endplates 16, 18. For example, FIG. 2B shows the endplates 16, 18 expanded in a parallel manner. In a second mode, the actuator screw 22 is held in place and the anterior actuator nut 24 is rotated or turned alone. This makes the anterior actuator 28 move alone which expands or contracts the anterior end of each endplate only and results in an increase in lordotic angle. For example, FIG. 2C shows the endplates 16, 18 expanded with an increased anterior height.

The expansion mechanism simplifies the expansion driver because the actuator screw 22 and the anterior actuator nut 24 need to be turned in the same direction for both lordotic and parallel expansion. In other designs, the components 22, 24 are rotated in opposite directions, for example, if the actuator screw needed to be turned clockwise for parallel expansion, the anterior actuator nut needed to be turned counterclockwise for lordotic expansion. This required either counterintuitive counter-clockwise motion for implant expansion, or a complex and expensive driver mechanism. Conversely, in this embodiment, the actuator screw 22 and the anterior actuator nut 24 are rotated in the same direction for both lordotic and parallel expansion, thereby simplifying the operation.

The implant 10 allows for continuous expansion and distraction over the range of that specific implant. This provides the ability to distract vertebral bodies to a desired height, but also collapse the device 10 for repositioning if desired. The implant 10 has the ability for the endplates 16, 18 to converge providing lordosis, while maintaining a large window for bone graft placement. By changing lordotic angulation, the implant 10 may match the patient's natural lordosis or be used to provide a specific lordosis at the level(s) treated.

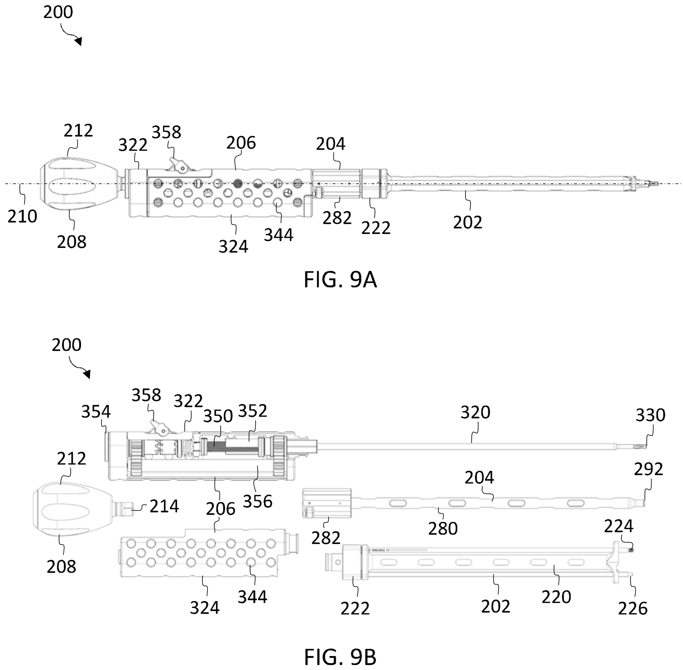

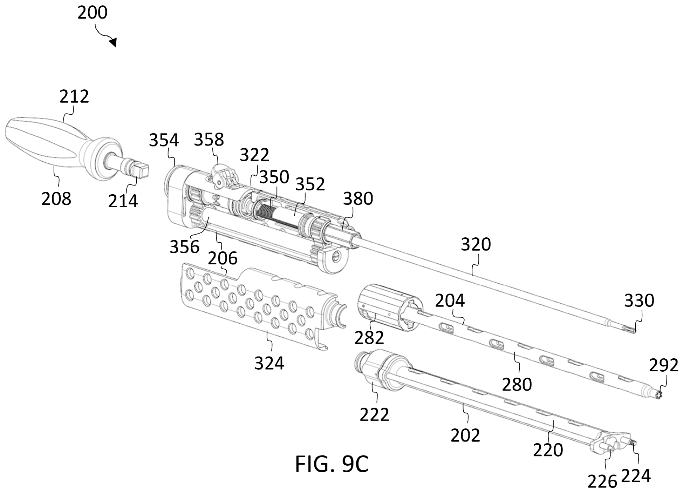

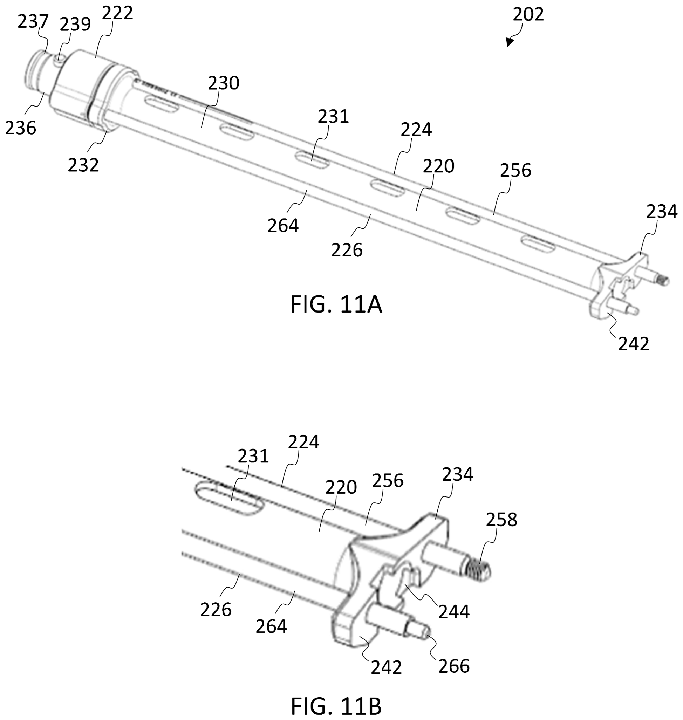

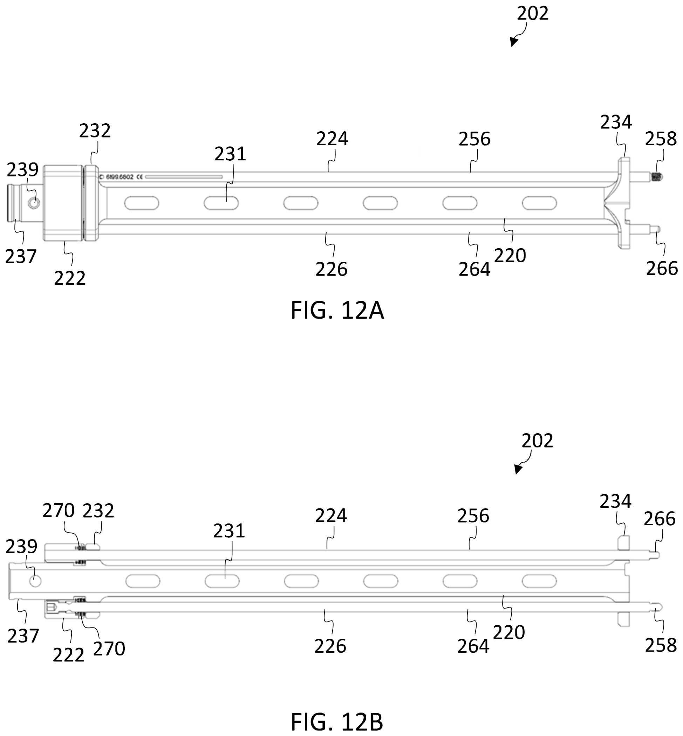

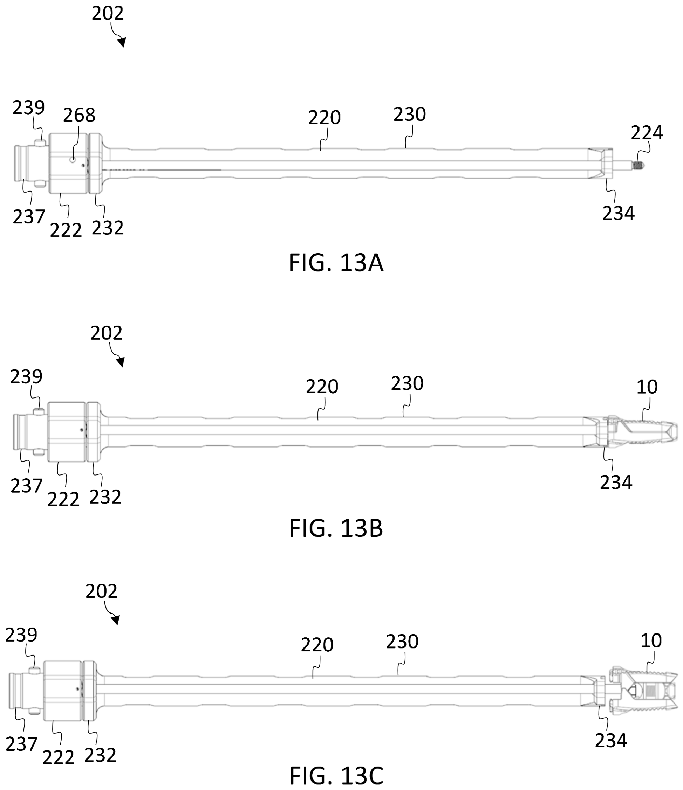

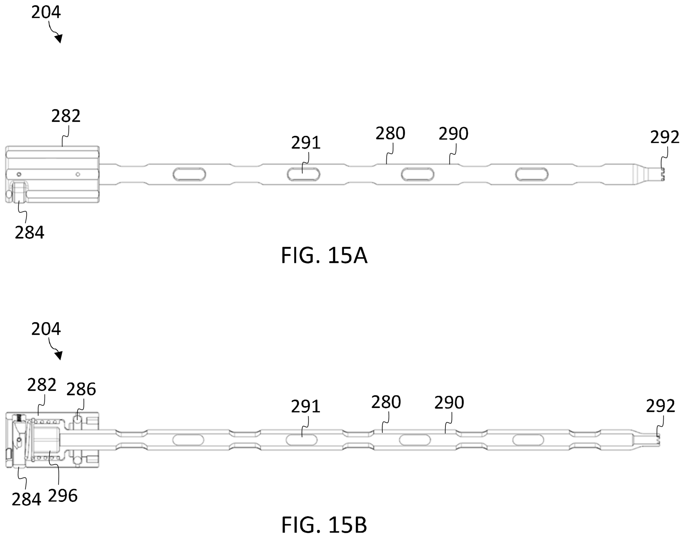

Turning now to FIGS. 9A-20B, an insertion and expansion instrument 200 is shown according to one embodiment. Instrument 200 is configured to insert the ALIF adjustable lordosis implant 10 into the disc space, for example, following a discectomy. The instrument 200 connects to the implant 10 in a zero-profile manner that allows the surgeon to visual the cage as it is inserted into a collapsed disc space. Once inserted, the instrument 200 allows the surgeon to independently control the parallel and lordotic expansion of the implant 10. The instrument 200 allows the surgeon to alternate between parallel and lordotic expansion with the flip of a switch 358. Indicators 420, 422 on the instrument 200 show measurements of the posterior and lordotic expansion. Quick-connectors allow for rapid assembly and disassembly of the various components without time-consuming or difficult assembly steps. The instrument 200 may be fully disassembled for cleaning without the need for additional tools.

As best seen in FIGS. 9A-9C, the instrument 200 may include four subassemblies: an insertion sub-assembly 202, an outer driver sub-assembly 204, an expansion driver sub-assembly 206, and a torque limiting handle 208, which align along an instrument axis 210. The insertion sub-assembly 202 is configured to attach directly to the implant 10 and may be used on its own to insert the implant 10 into the disc space or may be attached to the full inserter assembly 200. The outer driver sub-assembly 204 includes an outer driver 280 configured to engage with the anterior actuator nut 24 and transmit torque from the expansion sub-assembly 206. The expansion sub-assembly 206 includes an inner driver 320 configured to engage with the actuator screw 22 and control movement of the outer and inner drivers 280, 320, thereby controlling implant expansion. The torque limiting handle 208 includes a handle grip and an attachment interface 214, for example, for easy attachment and detachment to the expansion sub-assembly 206.

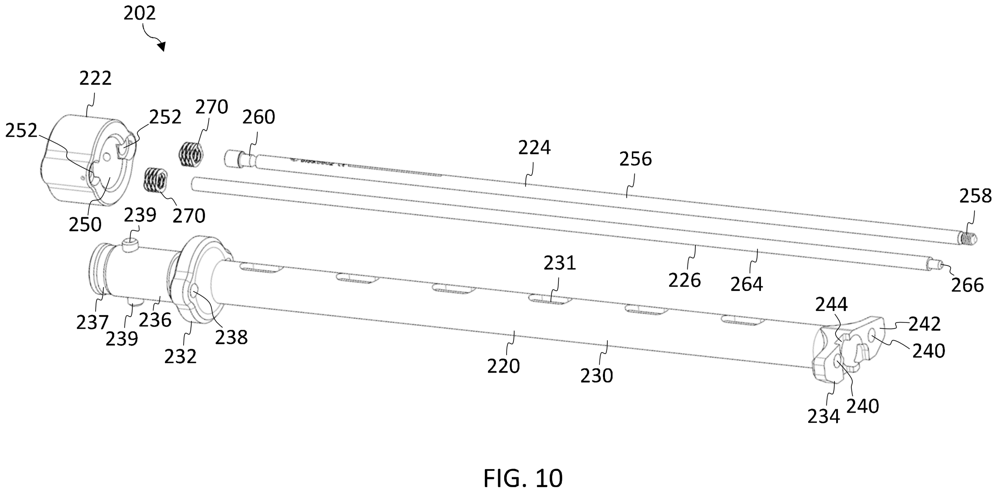

Turning now to FIGS. 10-13C, the insertion sub-assembly 202 is shown in more detail. The insertion sub-assembly 202 includes an outer tube 220, an insertion collar 222, a threaded rod 224, and a non-threaded rod 226. The outer tube 220 includes a hollow tube 230 with a proximal plate 232 and a distal plate 234 configured for securing the threaded and non-threaded rods 224, 226. The outer tube 230 may include one or more fenestrations 231, which may be in fluid communication with the longitudinal through bore. The insertion collar 222 is receivable onto a proximal portion 236 of the outer tube 230, which also connects to the outer driver sub-assembly 204. The proximal portion 236 of the outer tube 230 defines an annular groove 237, which forms a portion of the ball-seal connection to the outer driver assembly 204. The proximal portion 236 may also include one or more pins 239 (e.g., a pair of opposed pins 239), which interface with the outer driver assembly 204 to lock the implant 10 into any one of four, 90-degree offset positions based on surgeon preference. The keyed engagements 318 in the drive collar 282 allow four 90° orientations based on the surgeon's preference in handle ergonomics and visualization. The easy attachment/detachment from the inserter body allows for quick and easy orientation changes during surgery.

The proximal plate 232 may include an annular protrusion extending from the outer tube 230 defining a pair of bores 238 therethrough, which are configured to receive a proximal portion of the respective threaded and non-threaded rods 224, 226. The proximal plate 232 may protrude or bulge in the region of the bores 238 to accommodate the respective rods 224, 226. The distal plate 234 may include laterally extending wings extending from the outer tube 230 defining a pair of bores 240 therethrough, which receive a distal portion of the respective rods 224, 226. The respective bores 238, 240 may be aligned with one another and provided in parallel to receive the corresponding threaded and non-threaded rods 224, 226. The threaded and non-threaded rods 224, 226 extend between the proximal and distal plates 232, 234 on opposite sides of the outer tube 220 and may be aligned in parallel to one another. The distal plate 234 includes a distal face 242, which may contact the rear end 14 of the implant 10, when the instrument 200 is secured to the implant 10. The distal face 242 may define one or more notches or reliefs 244, for example, to provide clearance for the blocking screws 62 on the implant 10.

The insertion collar 222 includes an annular or ring-shaped body with a through opening 250 sized and dimensioned to receive the proximal portion 236 of the outer tube 220. The insertion collar 222 defines a pair of bores 252, which are configured to receive a proximal portion of the respective threaded and non-threaded rods 224, 226. These bores 250 similarly align with the corresponding bores 238, 240 through plates 232, 234. The insertion collar 222 may protrude or bulge on each side to accommodate the respective bores 252. The insertion collar 222 fits on the proximal portion 236 of the tube 220 and may abut against the proximal plate 232.

The insertion assembly 202 attaches securely to the implant 10 via threaded rod 224. The non-threaded rod 226 provides additional torsional stability. The threaded rod 224 may include an elongated non-threaded shaft 256 with a threaded distal tip 258. The threaded distal tip 258 may have a reduced diameter compared to shaft 256. The threaded distal tip 258 is configured to engage with the threaded bore 88 in the anterior actuator 28 of the implant 10. The threaded rod 224 may include a recessed shoulder 260 at its proximal end to interface with a pin 268 in the insertion collar 222. The non-threaded rod 226 may include an elongated non-threaded shaft 264 with a non-threaded distal tip 266. The non-threaded distal tip 266 may have a reduced diameter compared to shaft 264. The non-threaded distal tip 266 is configured to engage with the non-threaded bore 88 in the anterior actuator 28 of the implant 10. The threaded and non-threaded rods 224, 226 are connected back at the insertion collar 222. The threaded rod 224 is able to rotate, but constrained axially in the insertion collar 222 via pin 268 that mates with shoulder 260 on the threaded rod 224. The non-threaded rod 226 may be welded directly to the insertion collar 222 or otherwise secured to the insertion collar 222.

The insertion sub-assembly 202 may be spring-loaded to have a snug fit to the implant face during insertion. This allows impaction forces to be translated directly to the endplates 16, 18, but does not limit the articulation of the implant 10 as it would if it was tightened to the face of the inserter with a threaded connection. The insertion collar 222 may be spring-loaded backwards with two wave springs 270 that pull the implant 10 up against the face 242 of the inserter 202 when it is attached. The spring forces prevent the implant 10 from feeling loose on the inserter 202, but does not add resistance against the implant expansion, as would occur if the implant endplates 16, 18 were tightened to the face 242 of the inserter 202. This design ensures a snug fit to the inserter 202 without prohibiting the expansion mechanism.

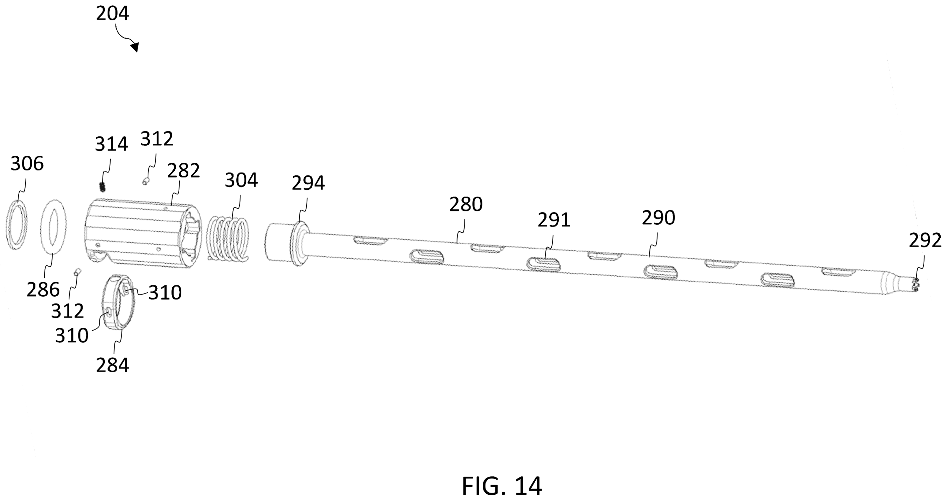

Turning now to FIGS. 14-16C, the outer driver sub-assembly 204 is shown in more detail. The outer driver sub-assembly 204 includes a spring-loaded outer driver 280 and a drive collar 282 which attaches the insertion sub-assembly 202 to the expansion sub-assembly 206. At one end of the drive collar 282, a spring-loaded button 284 may be used to connect the drive collar 282 to the expansion sub-assembly 206, and at the opposite end of the drive collar 282, a quick-connect ball seal 286 may be used to connect the drive collar 282 to the insertion sub-assembly 202.

The outer driver 280 includes a cannulated shaft or hollow tube 290, which allows the inner driver 320 to pass through it. The distal tip 292 may include a drive tip, for example, with driver teeth configured to engage with the anterior actuator nut 24. The distal drive tip 292 may be configured to mate with the driver engagement recess in the proximal end 150 of the anterior actuator nut 24 (e.g., castle nut drive feature) to apply torque to the anterior actuator nut 24. The drive tip 292 may have a reduced diameter compared to shaft 290. The tube 290 may include one or more fenestrations 291, which may be in fluid communication with the longitudinal through bore. The proximal end of the outer driver 280 includes an annular shoulder 294, which helps to retain the outer driver 280 in the drive collar 282. The outer driver 280 further defines an internal drive recess 296 (e.g., a hex recess) at the proximal-most end. The internal drive recess 296 may be sized and dimensioned to transmit torque as well as to allow the outer driver 280 to slide axially along the corresponding external hex driver 380 of the outer driver gear 352.

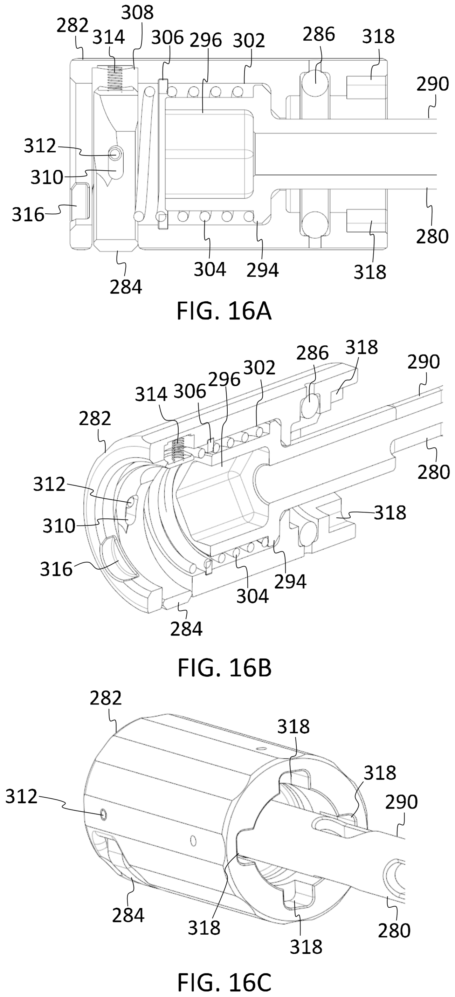

As best seen in FIGS. 16A-16C, the drive collar 282 has a cylindrical body defining an internal chamber 302 for receiving the proximal end of the outer driver 280. The outer driver 280 may be spring-loaded in the drive collar 282 via spring 304. The spring 304 may be positioned around the proximal portion of the shaft 290 and proximal to shoulder 294. The spring 304 may be secured via retaining ring 306. The outer driver 280 may be spring-loaded forward in order to maintain engagement with anterior actuator nut 24 or other outer drive feature on the implant 10. The spring-loaded outer driver 280 may have enough travel where if the driver teeth on the distal tip 292 do not line up with the implant 10, the outer driver 280 pushes back, and snaps forward into place once the outer driver 280 is turned. This avoids the need to perfectly align the drivers 280, 320 when attaching the implant 10.

The spring-loaded outer driver 280 includes spring 304 and retaining ring 306 to prevent disassembly. The outer driver 280 receives input torque from the expansion sub-assembly 206 via the internal drive recess 296 (e.g., a hex recess). The internal drive recess 296 not only transmits torque, but may also be long enough to allow the outer driver 280 to slide axially back along the corresponding external hex driver 380 when the instrument 200 is being attached to the implant 10.

The drive collar 282 houses a ball-seal connection 286 to the insertion sub-assembly 202 on one side, and a spring-loaded button connection 284 to the expansion sub-assembly 206 on the other. The back of the drive collar 282 contains spring-loaded button 284 and an alignment recess 316 to align to the expansion driver housing 322. The drive collar 282 retains the spring-loaded button 284 in a proximal cavity 308. The button 284 may include a ring-like structure or circular shape closely matching the outer profile of the drive collar 282. The button 284 may define bores 310, which are configured to receive respective pins 312 that secure the button 284 to the drive collar 282 on either side. The bores 310 may be elongated in length to allow for translation of the button 284. The cavity 308 within drive collar 282 also houses a small spring 314, which keeps a constant force pushing the button 284 out and keeping the outer driver assembly 204 locked to the expansion driver assembly 206. The button inner diameter may be chamfered on the leading edge to allow easy connection to the expansion driver housing 322. The alignment recess 316 may include a semicircular cutout or other suitably shaped recess along an inner surface of the proximal-most edge of the drive collar 282.

The front of the drive collar 282 houses the ball seal 286. The ball seal 286 may include a smooth ring that allows for even distribution of force ensuring a reliable seal when the insertion sub-assembly 202 is attached to the outer driver sub-assembly 204. When the proximal portion 236 of the insertion sub-assembly is seated into the distal end of the drive collar 282, the ball seal 286 fits into the groove 237 in the proximal portion 236 of the outer tube 220. As best seen in FIG. 16C, the drive collar 282 further defines four notches 318 into the distal-most face of the drive collar 282. The notches 318 are configured to interface with the pins 239 on the back of the insertion sub-assembly 202. The four notches 318 may be evenly spaced and symmetrically distributed about the circumference of the cavity to provide precise alignment functionality. The four notches 318 may be cut into the wall of the drive collar 282 extending radially outward at 90-degree intervals. Each notch 318 may have a uniform depth and width, which interact with the corresponding pins 239 on the proximal portion 236 of the outer tube 220 to lock the implant 10 into one of four possible offset orientations relative to the instrument 200.

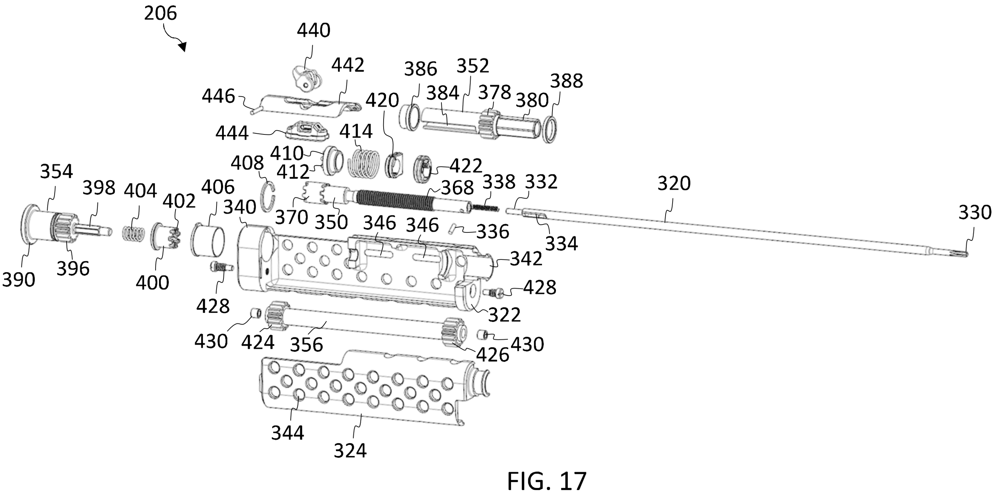

Turning now to FIGS. 17-20B, the expansion sub-assembly 206 is shown in more detail. The expansion sub-assembly 206 includes an inner driver 320 and a housing 322 with a cap 324, which contains the internal expansion mechanism 326 configured to control both the inner driver 320 and the outer driver 280. The inner driver 320 includes an elongated shaft, which is configured to extend through the outer driver 280. The inner driver 320 includes a distal drive tip 330, which is configured to engage with the actuator screw 22 of the implant 10. The distal drive tip 330 may be configured to mate with the instrument recess 146 in the proximal end 140 of the actuator screw 22 (e.g., a hex recess) to apply torque to the actuator screw 22. The drive tip 330 may have a reduced diameter compared to shaft 320. The proximal end 332 of the shaft 320 may also include a reduced diameter portion, which fits within the end of inner shaft 350. The inner driver 320 defines an elongated slot 334, which receives pin 336 to secure the inner driver 320 to the inner shaft 350. The inner driver 320 may be spring loaded to the inner shaft 350 via spring 338.

The housing 322 of the expansion driver sub-assembly 206 may include an elongated support structure with a closed proximal end 340 and an open distal end 342 for receiving the proximal end 332 of the inner driver 320. The housing 322 may be perforated, for example, with circular perforations 344 to reduce material weight while maintaining structural integrity. The housing 322 may be made from a light-weight material, such as aluminum. The housing 322 defines an inner cavity with guides, mounting points, or strategic locations configured to store and secure the components of the internal expansion mechanism 326. The housing 322 further defines one or more elongated windows 346 to observe the travel of one or more indicators 420, 422.

The housing 322 may be fully enclosed via cap 324, which may be removable once the outer driver sub-assembly 204 is removed. The cap 324 may include similar circular perforations 344. The removable cap 324 exposes the expansion mechanism 326 for cleaning. In an exemplary embodiment, none of these small components can be disassembled without specialized tooling to prevent them from being lost in sterile processing. This configuration may help to prevent unnecessary disassembly and/or lost parts in hospital sterile processing. The cap 324 may also help to prevent pinched fingers or gloves in the gearing during instrument use.

The internal expansion mechanism 326 may include an inner shaft 350 configured to engage with the inner driver 320, an outer driver gear 352 configured to engage with the outer driver 280, an input gear 354 which transmits torque from the handle 208 to the inner shaft 350 and/or outer driver gear 352, a parallel gear shaft 356 that transfers torque from the input gear 354 to the outer driver gear 352, and a switch assembly 358 that toggles between parallel and lordotic expansion functions.

The inner shaft 350 includes a proximal end 360 which interfaces with the input gear 354 and a distal end 362 that interfaces with the inner driver 320. The proximal end 360 defines a drive recess 364, which is configured to receive the hex drive 398 of the input gear 320. The distal end 362 defines a bore 366 configured to receive the proximal end 332 of the inner driver 320. A central portion of the inner shaft 350 may be threaded with external threads 368. The proximal end 360 includes a toothed member 370 with rear-facing teeth 372 configured to engage with teeth 402 on the parallel expansion gear 404 and front-facing teeth 374 configured to engage with teeth 412 on the anti-rotation gear 410.

The inner shaft 350 partially extends through the outer driver gear 352. The outer driver gear 352 includes an outer spur gear 378 that interfaces with a corresponding spur gear 426 on the parallel gear shaft 356 to thereby transmit torque from the parallel gear shaft 356 to the outer driver gear 352. The distal end of the outer driver gear 352 includes an outer driver, e.g., outer hex drive 380, which interfaces with the internal drive recess 296 (e.g., hex recess) on the outer driver 280, thereby transmitting torque to the outer driver 280. The proximal end of the outer driver gear 352 may include one or more longitudinal slots 384, thereby providing access to the inner shaft 350. The outer driver gear 352 may be retained with a bushing 386 and a retaining ring 388 positioned on opposite sides of the spur gear 378.

The input gear 354 transmits torque from the handle 208 to the inner shaft 350 and/or outer driver gear 352. The input gear 354 includes a proximal collar 390 that fits flush against the end of the housing 322, and defines a drive recess 392 configured for receiving the attachment interface 214 of the torque limiting handle 208. The handle 208 may be secured to the input gear 354 with a quick-connect ball seal 394 or other suitable quick-connect mechanism. The input gear 354 includes a spur gear 396 configured to interface with corresponding spur gear 424 on the parallel gear shaft 356 to thereby transmit torque to the outer driver gear 352. The distal end of the input gear 354 includes a shaft with an outer driver, e.g., outer hex drive 398 configured to transmit torque to the inner shaft 350 when engaged. The shaft of the input gear 354 is configured to pass through a parallel expansion gear 400, which has teeth 402 configured to engage with the rear-facing teeth 372 of the toothed member 370. The parallel expansion gear 400 may be biased via spring 404. The parallel expansion gear 400 slides along the hex drive 398 on the input gear 354 to allow the input torque to transfer to the inner shaft 350 when engaged. The input gear 354 spins in a bushing 406 in the housing 322 and is assembled with a retaining ring 408, such as a c-clip or c-shaped ring.

The anti-rotation gear 410 is positioned on the opposite side of the toothed member 370 to prevent rotation of the inner shaft 350. The anti-rotation gear 410 has teeth 412 configured to engage with the front-facing teeth 374 of the toothed member 370. The anti-rotation gear 410 is spring-loaded along the inner shaft 320 via spring 414. When the anti-rotation gear 410 slides backwards under spring load from the housing 322, the teeth 412 engage with the front-facing teeth 374 of the inner shaft, further preventing rotation of the inner shaft 350.

The inner shaft 350 may support one or more indicators 420, 422. The indicators 420, 422 may include rings that travel along the inner shaft 350 to indicate the degree of parallel and/or lordotic expansion. The indicators 420, 422 may have internal truncated threads that engage with the threaded portion 368 of the inner shaft 350. For example, the first expansion indicator 420 may be positioned along the threaded portion 368 of the inner shaft 350 to indicate parallel expansion, and a second expansion indicator 422 may be positioned along the outer driver gear 352 to indicate lordotic expansion. The indicators 420, 422 allow the surgeon to measure the parallel and lordotic expansion without the need for counting turns or measuring fluoroscopy images.

The parallel gear shaft 356 transmits torque from the input gear 354 to the outer driver gear 352. The parallel gear shaft 356 may include a shaft with a first spur gear 424 at one end and a second spur gear 426 at the opposite end of the shaft. The first spur gear 424 is configured to mesh with the spur gear 396 on the input gear 354 and the second spur gear 426 is configured to mesh with the spur gear 378 on the outer driver gear 352. The parallel gear shaft 356 may be secured to the housing 322 via fasteners 428, such as a set screw, at each end with bearings 430 configured to permit the parallel gear shaft 356 to rotate when transmitting torque from the input gear 354 to the outer driver gear 352.

The switch assembly 358 may include a switch 440, a mounting plate 442, a switch casing 444, and a pin 446. The switch 440 may include a toggle switch that pivots about pin 446. The switch 440 may have a forward position (e.g., parallel mode) and a back position (e.g., lordotic mode). The mounting plate 442 may form a portion of the housing 322 with a slot for receiving the switch casing 444. The switch casing 444 may include an elongated slot 448 for receiving the ends of pin 446. When the switch 440 is flipped, the switch casing 444 may travel to move the parallel expansion gear 400 and the anti-rotation gear 410 into or out of engagement with the inner shaft 350.

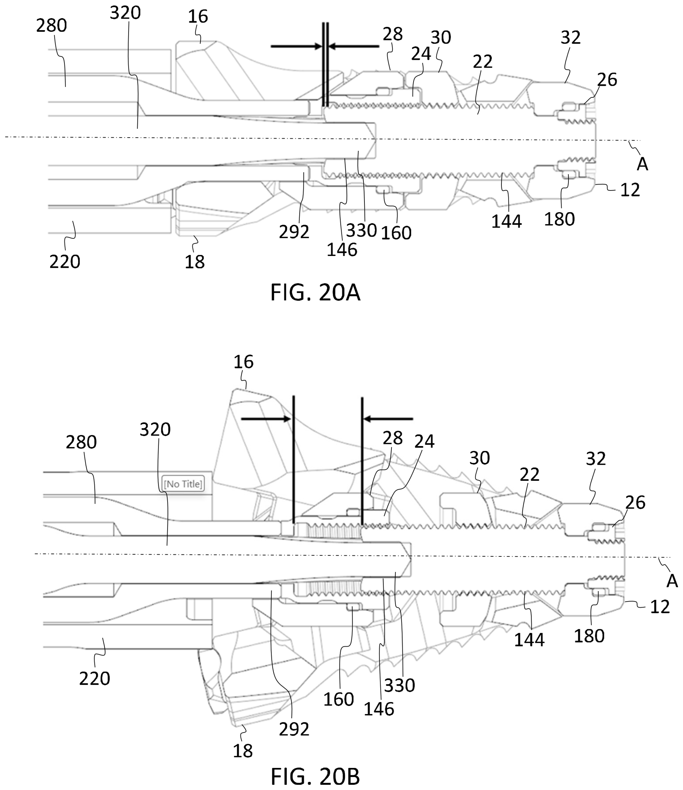

By way of example, FIGS. 20A-20B show expansion of the implant 10 when in parallel or lordotic mode. The inner driver 320 engages with the actuator screw 22 and the outer driver 280 engages with the anterior actuator nut 24. As best seen in FIG. 20A, in parallel mode, the outer and inner drivers 280, 320 are both turned together clockwise to expand the implant 10 in parallel. Because they are moving together simultaneously, the actuator screw 22 (inner driver 320) and outer driver 280 do not move relative to one another. As best seen in FIG. 20B, in lordosis mode, the outer driver 280 is turned clockwise, while the inner driver 320 is held still to expand in lordosis (or anterior expansion). The actuator screw 22 (inner driver 320) moves forward, away from the outer driver 280. When only the outer driver 280 is turning and the inner driver 320 is held still, the actuator screw 22 (inner driver 320) move forward creating a separation or distance between the actuator screw 22 (inner driver 320) and the outer driver 280.

All input torque may be delivered via the torque limiting handle 208 to the input gear 354. The input gear 354 may house ball seal 394 to retain the torque limiting handle 208. The end of the input gear 354 may also serve as an impaction cap to protect the aluminum housing 322 during impaction. The input gear 354 spins in PEEK bushing 406 in the housing 322 and is assembled with retaining ring 408. The spur gear 396 on the input gear 354 interfaces with the spur gear 424 on the parallel shaft 356. The spur gear 426 on the opposite end of the parallel shaft 356 interfaces with the spur gear 378 on the outer driver gear 352, which drives the outer shaft 280 via the hex drive 380 on its far end. This gear train from the input gear 354, through the parallel shaft 356, to the outer driver gear 352 may be permanently linked such that any time the handle 208 is turned the outer shaft 280 is always turning. Parallel vs. lordotic expansion may be determined by if the inner shaft 320 is turned along with the outer shaft 280 (parallel expansion), or if the inner shaft 320 is held still (lordotic expansion).

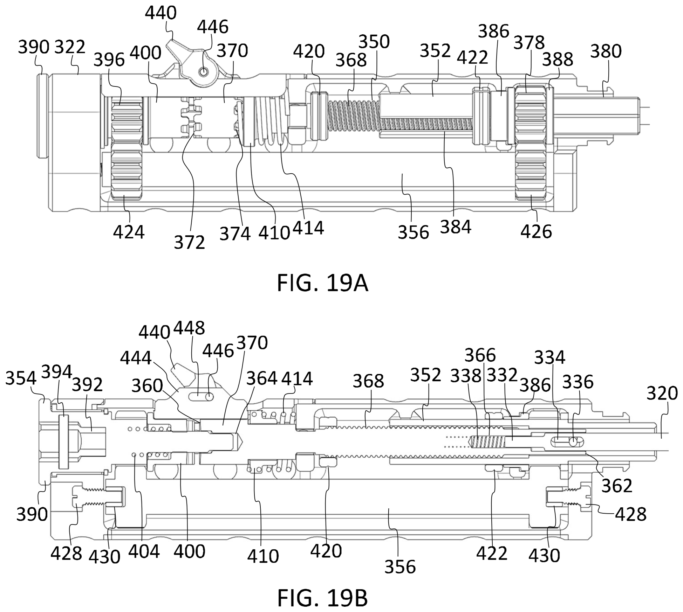

Parallel and lordotic expansion modes may be activated by flipping the switch 440. When the switch 440 is back, the instrument 200 is in lordotic mode, and when the switch 440 is forward, the instrument 200 is in parallel expansion mode. FIGS. 19A-19B show the switch 440 backward with the instrument 200 in lordotic expansion mode. As described herein, the back-half of the inner shaft 350 has both front and back-facing teeth 372, 374 along toothed member 370. These teeth 372, 374 may act as a clutch plate to either transmit rotation to the inner shaft 350, or counter-torque the inner shaft 350 against rotation. If the inner shaft is not counter-torqued, the implant 10 can expand in parallel when turning the outer driver 280 alone due to the frictional forces in the implant 10. The spring loaded clutch plate 370 engages this counter torque automatically when switched to lordotic expansion mode.

When in parallel expansion mode, the switch 440 is flipped forward. The switch 440 pushes the anti-rotation gear 410 forward, disengaging the teeth 412 from the teeth 374 of the inner shaft 350. Simultaneously, the parallel expansion gear 400 shifts forward with the switch 440 under spring-load from the input gear 390, engaging teeth 402 with the teeth 372 of the inner shaft 350. The parallel expansion gear 400 slides along hex 398 on the input gear 354, allowing the input torque to transfer to the inner shaft 350, as well as to the parallel gear shaft 356, outer driver gear 352, and ultimately to the outer driver 280, for parallel expansion.

When in lordotic expansion mode, the switch 440 is flipped back. The switch 440 pushes the parallel expansion gear 400 backwards, disengaging the teeth 402 with the teeth 372 of the inner shaft 350. This prevents any input torque from engaging the inner shaft 350. Simultaneously, the anti-rotation gear 410 slides backwards under spring load from the housing 322, engaging teeth 412 with the teeth 374 of the inner shaft 350. The anti-rotation gear 410 may also have a flat or keying feature that mates with the housing 322 and prevents it from rotating. This counter-torques the inner shaft 350 from being able to rotate, thereby providing for the lordotic expansion.

The front-half of the inner driver 320 may be spring-loaded forward and pinned inside the back-half of the inner shaft 350. The inner driver 320 may be spring-loaded in order to allow the inner driver tip 330 to easily snap into place if misaligned, and to ensure the inner driver tip 330 remains engaged with the implant actuator screw 22 as the implant 10 goes from a parallel to a lordotic position and the actuator screw 22 moves away from the outer driver 280.

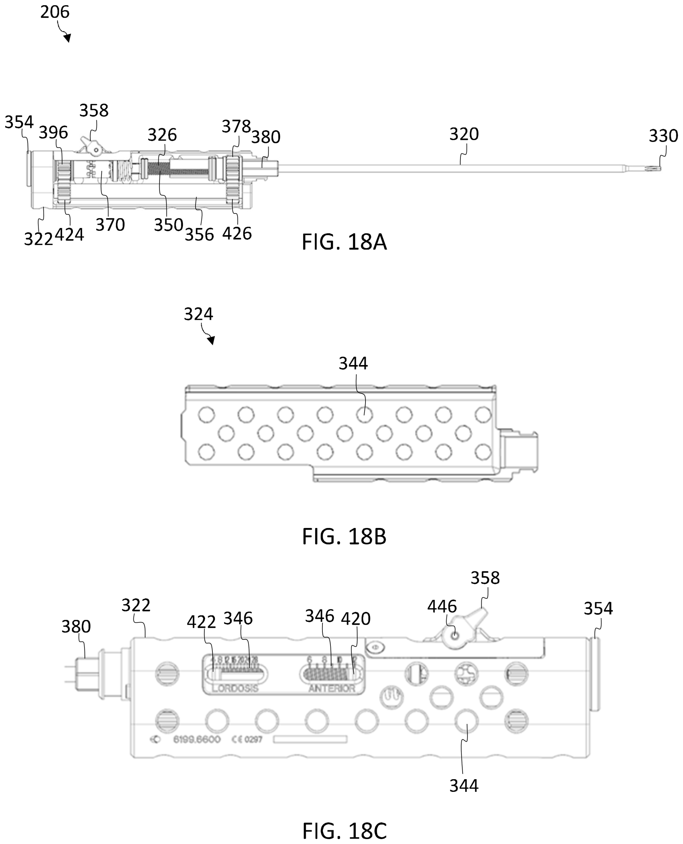

As best seen in FIG. 18C, the expansion driver housing 322 may include two windows 346 to observe the travel of the parallel expansion indicator 420 and the lordosis indicator 422. Both of these indicators 420, 422 may be threaded onto the back-half of the inner shaft 350. The parallel expansion indicator 420 may have a flat that prevents rotation relative to the housing 422 such that the indicator 420 translates along the inner shaft 350 any time the inner shaft 350 is rotated. The inner shaft 350 is only rotated during parallel expansion; therefore, this translation can be used to indicate the amount of parallel expansion in the implant 10. The lordosis indicator 422 slides over the outer driver gear 352 and has threads that reach through the slots 384 in the outer driver gear 352 to engage with the threads 368 of the inner shaft 350. The lordosis indicator 422 translates along the inner shaft 350 when there is relative rotation between the inner and outer shafts 350, 352. This only occurs when the outer driver 280 is rotated for lordotic expansion, and as such, this translation can be used to indicate the amount of lordosis in the implant 10. Both indicators 420, 422 may include truncated threads and a spring-cut to allow the indicators 420, 422 to skip over the threads 368 of the inner shaft 350 when it reaches the end of travel. This may help to prevent the expansion sub-assembly 206 from binding up in the event that the indicators 420, 422 are not reset to zero prior to use. In other words, the indicators 420, 422 ability to skip over the threads when they hit the end of travel avoids the inserter binding up and preventing expansion if the surgical team forgets to reset the indicators 420, 422 prior to the procedure.

The user is able to instantly alternate between parallel and lordotic expansion by merely flipping the switch 440. This allows for surgeons to dial in a precise fit of the implant 10 to the patient anatomy, thereby avoiding additional cognitive load on the surgeon from a complex instrument. The internal expansion mechanism 326 allows parallel and lordotic expansion to be performed by turning the handle 208 in the same direction. Both of the inner and outer drivers 280, 320 are spring loaded to allow for easy engagement to the implant 10. This reduces surgical time trying to line up the drivers 280, 320 to the implant 10.

The instrument 200 breaks down into multiple pieces to allow access to all difficult-to-clean areas. The instrument 200 fully disassembles without the need for tools and the disassembled components are sizable enough to ensure that no components are lost or damaged during the cleaning process. The modular design also ensures that the instrument 200 can be easily repaired without having to scrap large portions of an expensive instrument.

Although the invention has been described in detail and with reference to specific embodiments, it will be apparent to one skilled in the art that various changes and modifications can be made without departing from the spirit and scope of the invention. Thus, it is intended that the invention covers the modifications and variations of this invention provided they come within the scope of the appended claims and their equivalents. It is expressly intended, for example, that all components of the various devices disclosed above may be combined or modified in any suitable configuration.

The invention could be inter alia defined by the following examples:
1. An expandable implant comprising: an upper endplate and a lower endplate configured to engage adjacent vertebrae; and an actuator assembly for expanding the upper and lower endplates to independently control anterior and posterior heights of the implant, the actuator assembly including a moveable anterior actuator, a moveable posterior actuator, and a stationary posterior actuator positioned between the upper and lower endplates, an actuator screw threads into an anterior actuator nut located in the moveable anterior actuator and a posterior actuator nut located in the stationary posterior actuator, the actuator screw threads through the moveable posterior actuator to translate the moveable posterior actuator, the upper and lower endplates interface with the moveable anterior actuator, moveable posterior actuator, and stationary posterior at complimentary mating ramps that slidably engage with one another to allow for expansion of the upper and lower endplates, the mating ramps include single point of contact ramps and full contact ramps, wherein when the actuator screw and anterior actuator nut are turned together, the moveable posterior actuator moves toward the stationary posterior actuator, forcing the upper and lower endplates apart resulting in parallel expansion, and when only the anterior actuator nut is turned, the moveable anterior actuator moves alone to increase the anterior height, resulting in an increase in lordotic angle.
2. The expandable implant of example **1,** wherein the single point of contact ramps include a single point or edge of contact along one ramp surface that makes contact with a corresponding ramp surface
3. The expandable implant of example **1,** wherein the full contact ramps include constant continuous contact between the ramped surfaces, thereby carrying a majority of lifting force during expansion.
4. The expandable implant of example **1,** wherein the single point of contact ramps are located near lateral sides of the implant and the full contact ramps are located in between the single point of contact ramps close to a midline of the implant.
5. The expandable implant of example **1,** wherein the anterior actuator defines a pair of pin holes configured to receive limit pins that retain the anterior actuator nut in the anterior actuator.
6. The expandable implant of example 5, wherein the pin holes define non-threaded vertical slots that are generally perpendicular to a longitudinal axis of the implant
7. The expandable implant of example 5, wherein the limit pins include a solid body with an L-shaped notched end and an enlarged base at the opposite end.
8. The expandable implant of example 5, wherein the limit pins limit the travel of the anterior actuator assembly by contacting surfaces on the upper and lower endplates when the implant is at its maximum expansion or lordosis.
9. An insertion and expansion instrument comprising: an insertion sub-assembly configured to attach directly to an implant, the insertion sub-assembly having an outer tube with a threaded rod and non-threaded rod on opposite sides of the outer tube, and an insertion collar for receiving the outer tube, threaded rod, and non-threaded rod; an outer driver sub-assembly having an outer driver positionable through the outer tube and a drive collar that attaches to the outer tube; an expansion sub-assembly having an inner driver positionable through the outer driver and a housing with a removable cap containing an internal expansion mechanism configured to control both the inner driver and the outer driver, the internal expansion mechanism including an inner shaft configured to engage with the inner driver, an outer driver gear configured to engage with the outer driver, an input gear which transmits torque to the inner shaft and/or outer driver gear, and a switch assembly that switches between parallel and lordotic expansion modes; and a torque limiting handle configured to transmit torque to the input gear
10. The insertion and expansion instrument of example 9, wherein in parallel expansion mode, the expansion mechanism rotates the inner driver and outer driver simultaneously, and in lordotic expansion mode, only the outer driver rotates while the inner driver remains stationary.
11. The insertion and expansion instrument of example 9, wherein the internal expansion mechanism includes a parallel expansion gear defining teeth, an anti-rotation gear defining teeth, and the inner shaft includes a toothed member with front-facing teeth configured to interface with the teeth of the anti-rotation gear and rear-facing teeth configured to interface with the teeth of the parallel expansion gear.
12. The insertion and expansion instrument of example 11, wherein the switch assembly includes a switch, a mounting plate attached to the housing, a switch casing positioned in the mounting plate and having an elongated slot, and a pin connecting the switch to the elongated slot of the switch casing, wherein when the switch is flipped, the switch casing travels to move the parallel expansion gear and the anti-rotation gear into or out of engagement with the toothed member of inner shaft.
13. The insertion and expansion instrument of example 9, wherein the internal expansion mechanism includes a parallel gear shaft aligned in parallel to the inner shaft, the parallel gear shaft includes spur gears at each end, the input gear includes a spur gear which interfaces with the spur gear at one end of the parallel gear shaft, and the outer driver gear includes a spur gear which interfaces with the spur gear at the other end of the parallel gear shaft to thereby transfer motion from the input shaft, through the parallel gear shaft, and to the outer driver gear.
14. The insertion and expansion instrument of example 13, wherein a parallel expansion indicator is positioned along a threaded portion of the inner shaft, and a lordotic expansion indicator is positioned along the outer driver gear, the housing defines a pair of windows to view travel of the respective indicators, thereby representing an amount of parallel and/or lordotic expansion.
15. The insertion and expansion instrument of example 9, wherein the inner and outer drivers are spring-loaded forward in order to maintain engagement with the implant.
16. The insertion and expansion instrument of example 9, wherein the drive collar defines four notches at 90-degree intervals, which are configured to interface with pins on the outer tube of the insertion sub-assembly, to thereby lock the implant into one of four possible offset orientations.
17. The insertion and expansion instrument of example 9, wherein the outer tube includes a proximal plate defining a pair of bores and a distal plate defining a pair of bores, wherein the respective bores secure the threaded and non-threaded rods to the outer tube, and the distal plate includes a distal face configured to contact the implant when distal ends of the threaded and non-threaded rods engage with the implant.
18. A method of spinal fixation comprising: attaching an insertion and expansion instrument to an expandable intervertebral implant, the expandable intervertebral implant includes upper and lower endplates and an actuator assembly including a moveable anterior actuator, a moveable posterior actuator, and a stationary posterior actuator positioned between the upper and lower endplates, an actuator screw threads into an anterior actuator nut located in the moveable anterior actuator and a posterior actuator nut located in the stationary posterior actuator, the actuator screw threads through the moveable posterior actuator to translate the moveable posterior actuator, and the insertion and expansion instrument includes an insertion sub-assembly having an outer tube with a threaded rod and a non-threaded rod on opposite sides of the outer tube, which engage with the moveable anterior actuator, an outer driver sub-assembly having an outer driver positionable through the outer tube, an expansion sub-assembly having an inner driver positionable through the outer driver and a housing with a removable cap containing an internal expansion mechanism configured to control both the inner driver and the outer driver, and a torque limiting handle configured to transmit torque to the internal expansion mechanism; inserting the expandable intervertebral implant into a disc space between adjacent vertebrae; and expanding the implant by engaging the outer driver with the anterior actuator nut and the inner driver with the actuator screw and via the insertion and expansion instrument either: (1) rotating the inner driver and outer driver simultaneously to rotate the actuator screw and the anterior actuator nut together to expand the upper and lower endplates in parallel expansion; or (2) rotating only the outer driver to rotate the anterior actuator nut to expand the upper and lower endplates in lordotic expansion.
19. The method of example 18, wherein the inner driver and outer driver rotate simultaneously in the same direction to turn the actuator screw and anterior actuator nut simultaneously
20. The method of example 18 further comprising inserting bone anchors through sockets in the upper and lower endplates and into the adjacent vertebrae, and rotating blocking screws in the upper and lower endplates to cover the bone anchors and prevent the bone anchors from backing out.

## Claims

1. An expandable implant comprising:
an upper endplate and a lower endplate configured to engage adjacent vertebrae; and
an actuator assembly for expanding the upper and lower endplates to independently control anterior and posterior heights of the implant, the actuator assembly including a moveable anterior actuator, a moveable posterior actuator, and a stationary posterior actuator positioned between the upper and lower endplates, an actuator screw threads into an anterior actuator nut located in the moveable anterior actuator and a posterior actuator nut located in the stationary posterior actuator, the actuator screw threads through the moveable posterior actuator to translate the moveable posterior actuator, the upper and lower endplates interface with the moveable anterior actuator, moveable posterior actuator, and stationary posterior at complimentary mating ramps that slidably engage with one another to allow for expansion of the upper and lower endplates, the mating ramps include single point of contact ramps and full contact ramps,
wherein when the actuator screw and anterior actuator nut are turned together, the moveable posterior actuator moves toward the stationary posterior actuator, forcing the upper and lower endplates apart resulting in parallel expansion, and when only the anterior actuator nut is turned, the moveable anterior actuator moves alone to increase the anterior height, resulting in an increase in lordotic angle.

2. The expandable implant of claim **1,** wherein the single point of contact ramps include a single point or edge of contact along one ramp surface that makes contact with a corresponding ramp surface.

3. The expandable implant of claim 1 or 2, wherein the full contact ramps include constant continuous contact between the ramped surfaces, thereby carrying a majority of lifting force during expansion.

4. The expandable implant of any one of the preceding claims, wherein the single point of contact ramps are located near lateral sides of the implant and the full contact ramps are located in between the single point of contact ramps close to a midline of the implant.

5. The expandable implant of any one of the preceding claims, wherein the anterior actuator defines a pair of pin holes configured to receive limit pins that retain the anterior actuator nut in the anterior actuator and wherein preferably the pin holes define non-threaded vertical slots that are generally perpendicular to a longitudinal axis of the implant and wherein preferably the limit pins include a solid body with an L-shaped notched end and an enlarged base at the opposite end.

6. The expandable implant of claim 5, wherein the limit pins limit the travel of the anterior actuator assembly by contacting surfaces on the upper and lower endplates when the implant is at its maximum expansion or lordosis.

7. An insertion and expansion instrument comprising:
an insertion sub-assembly configured to attach directly to an implant, the insertion sub-assembly having an outer tube with a threaded rod and non-threaded rod on opposite sides of the outer tube, and an insertion collar for receiving the outer tube, threaded rod, and non-threaded rod;
an outer driver sub-assembly having an outer driver positionable through the outer tube and a drive collar that attaches to the outer tube;
an expansion sub-assembly having an inner driver positionable through the outer driver and a housing with a removable cap containing an internal expansion mechanism configured to control both the inner driver and the outer driver, the internal expansion mechanism including an inner shaft configured to engage with the inner driver, an outer driver gear configured to engage with the outer driver, an input gear which transmits torque to the inner shaft and/or outer driver gear, and a switch assembly that switches between parallel and lordotic expansion modes; and
a torque limiting handle configured to transmit torque to the input gear.

8. The insertion and expansion instrument of claim 7, wherein in parallel expansion mode, the expansion mechanism rotates the inner driver and outer driver simultaneously, and in lordotic expansion mode, only the outer driver rotates while the inner driver remains stationary.

9. The insertion and expansion instrument of claim 7 or 8, wherein the internal expansion mechanism includes a parallel expansion gear defining teeth, an anti-rotation gear defining teeth, and the inner shaft includes a toothed member with front-facing teeth configured to interface with the teeth of the anti-rotation gear and rear-facing teeth configured to interface with the teeth of the parallel expansion gear.

10. The insertion and expansion instrument of claim 9, wherein the switch assembly includes a switch, a mounting plate attached to the housing, a switch casing positioned in the mounting plate and having an elongated slot, and a pin connecting the switch to the elongated slot of the switch casing, wherein when the switch is flipped, the switch casing travels to move the parallel expansion gear and the anti-rotation gear into or out of engagement with the toothed member of inner shaft.

11. The insertion and expansion instrument of any one of claims 7-10, wherein the internal expansion mechanism includes a parallel gear shaft aligned in parallel to the inner shaft, the parallel gear shaft includes spur gears at each end, the input gear includes a spur gear which interfaces with the spur gear at one end of the parallel gear shaft, and the outer driver gear includes a spur gear which interfaces with the spur gear at the other end of the parallel gear shaft to thereby transfer motion from the input shaft, through the parallel gear shaft, and to the outer driver gear.

12. The insertion and expansion instrument of claim 11, wherein a parallel expansion indicator is positioned along a threaded portion of the inner shaft, and a lordotic expansion indicator is positioned along the outer driver gear, the housing defines a pair of windows to view travel of the respective indicators, thereby representing an amount of parallel and/or lordotic expansion.

13. The insertion and expansion instrument of any one of claims 7-12, wherein the inner and outer drivers are spring-loaded forward in order to maintain engagement with the implant.

14. The insertion and expansion instrument of any one of claims 7-13, wherein the drive collar defines four notches at 90-degree intervals, which are configured to interface with pins on the outer tube of the insertion sub-assembly, to thereby lock the implant into one of four possible offset orientations.

15. The insertion and expansion instrument of any one of claims 7-14, wherein the outer tube includes a proximal plate defining a pair of bores and a distal plate defining a pair of bores, wherein the respective bores secure the threaded and non-threaded rods to the outer tube, and the distal plate includes a distal face configured to contact the implant when distal ends of the threaded and non-threaded rods engage with the implant.
